(19)

Europäisches Patentamt

European Patent Office

Office européen des brevets

(11) **EP 4 140 475 A1**

(12) **EUROPEAN PATENT APPLICATION**

(43) Date of publication:
01.03.2023 Bulletin 2023/09

(21) Application number: 21193839.4

(22) Date of filing: 30.08.2021

(51) International Patent Classification (IPC):
$A61K\ 9/00$ *(2006.01)*    $A61K\ 9/08$ *(2006.01)*
$A61K\ 9/127$ *(2006.01)*    $A61K\ 9/51$ *(2006.01)*
$A61K\ 39/12$ *(2006.01)*    $A61P\ 31/14$ *(2006.01)*
$A61P\ 31/16$ *(2006.01)*

(52) Cooperative Patent Classification (CPC):
A61K 9/0019; A61K 9/08; A61K 9/127;
A61K 9/1277; A61K 9/5123; A61K 39/12;
A61P 31/14; A61P 31/16; A61P 31/20;
A61K 2039/55555; C12N 2730/10134;
C12N 2770/20034; C12N 2770/24134

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: Max-Planck-Gesellschaft zur
Förderung der
Wissenschaften e.V.
80539 München (DE)

(72) Inventor: **The designation of the inventor has not yet been filed**

(74) Representative: **Arth, Hans-Lothar**
**ABK Patent Attorneys**
**Jasminweg 9**
**14052 Berlin (DE)**

Remarks:
The complete document including Reference
Table(s) and the Sequence Listing(s) can be
downloaded from the EPO website

(54) **SYNTHETIC VIRUS-LIKE LIPOPARTICLES**

(57)    The present invention is directed to non-infectious synthetic lipoparticles. Said lipoparticles comprise a cell surface receptor binding Spike protein ectodomain on their surface, which renders the lipoparticles particularly useful for studying the binding and/or retention of said lipoparticles to cells.

**Figure 1**

## Description

The present invention is directed to non-infectious synthetic lipoparticles. Said lipoparticles comprise a cell surface receptor binding Spike protein ectodomain on their surface, which renders the lipoparticles particularly useful for studying the binding and/or retention of said lipoparticles to cells.

## Background of the invention

Human pathogens enter their host cells, and eventually kill or weaken them, using cellular receptors. In nearly all cases, these cellular receptors are membrane proteins on the cell surface, i.e. cell surface receptors. Identifying these membrane proteins and linking infectious agents to their receptors offers direct insight into disease pathogenesis. For example, SARS-CoV-2 infection is initiated by binding of the virus to the target receptor angiotensin converting enzyme 2 (ACE2), mediated by the trimeric Spike (S) glycoprotein. Also, HIV uses a fusion co-receptor, typically either the chemokine receptors CXCR4 or CCR5. Preventing receptor binding is a common strategy for treating or preventing pathogen infection by inhibition of viral entry.

Despite their importance, surface proteins present a unique set of challenges for ligand binding and drug discovery studies because they require a lipid environment to maintain native structure. Consequently, interaction studies involving membrane proteins typically use whole cells or vesicles derived from cell membranes, where the protein of interest is but a minor component, making both adequate sensitivity and specificity more difficult to achieve. Living cells are cumbersome to grow, must be maintained in a high protein (serum) environment, and present a moving target as receptors are internalized, altered by intracellular events, and recycled. Cells also have severe limitations in their application to biosensors and other microfluidic devices, most notably in their size, sensitivity to environmental conditions, and heterogeneous cell surface. Membrane vesicles, prepared by mechanically disrupting cells (Top-down approach), are a common source of membrane protein for many drug screens currently conducted. However, membrane vesicles are heterogeneous, impure, and not particularly stable. The receptors within them may be misoriented, a minor component of total protein, and derived from intracellular organelles. Thus, pseudovirus approaches as well as in *in vitro* protein binding assays often do not reproduce the biophysical features of the natural pathogen (virion, cell), which are essential for assessing particulate and multivalent binding effects.

Moreover, the techniques currently used to study pathogen receptor interactions are generally slow and laborious. In many cases, such techniques require transfection of individual receptors, the growth of cells, and the use of the pathogen in its infectious form, thereby requiring at least a safety level 2 laboratory environment. Also, protein interactions are detected using immunoprecipitation, Western blotting, or radiolabeled proteins.

The global COVID-19 pandemic, caused by the enveloped severe acute respiratory syndrome-coronavirus 2 (SARS-CoV-2), creates an urgent unmet need for effective antiviral strategies. SARS-CoV-2 infection is initiated by binding of the virus to the target receptor angiotensin converting enzyme 2 (ACE2), mediated by the trimeric Spike (S) glycoprotein. Initial binding is achieved by the RBD within the S1 portion of S, while S2 induces fusion of the viral envelope with the target cell membrane. S is the major focus of COVID-19 vaccine development, the primary target for neutralizing antibodies and the main diagnostic antigen for SARS-CoV-2 infections. Structural analyses of S revealed substantial conformational rearrangements of RBD during the infection process. In a closed conformation, the RBDs lie flat on the S surface and are therefore less accessible for ACE2 binding. In open conformations, at least one RBD erects from S to expose the receptor binding motif, enabling ACE2 binding. Each monomer within the S trimer can undergo a closed-to-open transition and consecutive priming by ACE2. On intact SARS-CoV-2 virions, ~31% of S trimers are found in a closed state, ~55% with one open RBD and ~14% with two open RBDs. Molecular mechanisms that modulate S open-to-closed equilibrium and their implications for SARS-CoV-2 infection and immune evasion remain poorly understood. Exogenous control of this equilibrium, however, could greatly benefit COVID-19 therapy development and promote prospective pandemic prevention.

Thus, there is a need for the development of an improved technique that enables studying of interactions between pathogens, particularly viruses, and living cells on a molecular level under low-biosafety constraints and with little effort.

Therefore, the objective of the present invention lies in the provision of synthetic particles that mimic pathogens, particularly viruses, that can be prepared under low-biosafety constraints and that are particularly useful for studying the binding and/or retention to living cells.

The objective of the present invention is solved by the teaching of the independent claims. Further advantageous features, aspects and details of the invention are evident from the dependent claims, the description, the figures, and the examples of the present application.

**Brief description of the invention**

It was found that synthetic lipoparticles comprising a lipid bilayer decorated with cell receptor binding Spike protein ectodomain are particularly beneficial for assessing receptor binding. The so formed synthetic lipoparticles are molecularly defined and suitable for systematic analysis of cell attachment effects.

The synthetic lipoparticles can be produced *in vitro* in a bottom-up approach from small unilamellar vesicles. Due to the bottom-up approach the constitution of the lipid bilayer in regard of the lipid components, the Spike protein and particle size, can be adjusted precisely to mimic the biophysical features of various natural pathogens, such as viruses or virions, thereby allowing for integrated assessment of particulate and multivalent binding effects between the pathogen and living cells.

Moreover, the synthetic lipoparticles according to the invention lack genetically encoded information, so that no higher biosafety standards have to be met, which facilitates the production and use of the synthetic lipoparticles.

Thus, the present invention is directed to a non-infectious synthetic lipoparticle comprising: a lipid bilayer comprising

- between 40 and 85 mol percent of a phosphatidylcholine as neutral amphiphile,
- at least one anionic lipid, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering.

The synthetic lipoparticles of the present invention do not contain infectious nucleic acids, such as DNA or RNA, wherein infectious refers to nucleic acids which, upon being taken up by a permissive host cell, are capable of producing multiple, e.g., one or more copies of the same nucleic acid (replicating) and the nucleic acid represent a complete infectious virus particle.

In other words, the present invention is directed to a synthetic lipoparticle comprising: a lipid bilayer comprising

- between 40 and 85 mol percent of a phosphatidylcholine as neutral amphiphile,
- at least one anionic lipid, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering, and wherein the lipoparticle does not comprise an infectious nucleic acid.

The synthetic particles of the present invention mainly consist of a lipid bilayer and are therefore considered as lipoparticles. The lipid bilayer forms a shell or a sphere in the nanometer range similar to the outer layer of viruses or virions, the viral envelope, so that the synthetic lipoparticles of the present invention exhibit biophysical properties similar or identical to natural viruses or virions. In this regard, the lipoparticles are virus-like or viral particles or minimal virions.

In other words, the present invention is directed to a non-infectious synthetic virus-like particle comprising: a lipid bilayer comprising

- between 40 and 85 mol percent of a phosphatidylcholine as neutral amphiphile,
- at least one anionic lipid, and
- at least 1 mol percent of a lipid-bound Spike protein ectodomain, wherein the Spike protein ectodomain binds to a cell surface receptor,

wherein the virus-like particle has a diameter between 20 nm and 300 nm as measured by dynamic light scattering.

Reworded, the present invention is directed to a non-infectious synthetic viral particle comprising: a lipid bilayer comprising

- between 40 and 85 mol percent of a phosphatidylcholine as neutral amphiphile,
- at least one anionic lipid, and
- at least 1 mol percent of a lipid-bound Spike protein ectodomain, wherein the Spike protein ectodomain binds to a cell surface receptor,

wherein the viral particle has a diameter between 20 nm and 300 nm as measured by dynamic light scattering.

Reworded, the present invention is directed to a non-infectious synthetic minimal virion comprising: a lipid bilayer comprising

- between 40 and 85 mol percent of a phosphatidylcholine as neutral amphiphile,
- at least one anionic lipid, and
- at least 1 mol percent of a lipid-bound Spike protein ectodomain, wherein the Spike protein ectodomain binds to a cell surface receptor,

wherein the minimal virion has a diameter between 20 nm and 300 nm as measured by dynamic light scattering.

Reworded, the present invention is directed to a non-infectious synthetic liposome comprising: a lipid bilayer comprising

- between 40 and 85 mol percent of a phosphatidylcholine as neutral amphiphile,
- at least one anionic lipid, and
- at least 1 mol percent of a lipid-bound Spike protein ectodomain, wherein the Spike protein ectodomain binds to a cell surface receptor,

wherein the liposome has a diameter between 20 nm and 300 nm as measured by dynamic light scattering.

Reworded, the present invention is directed to a non-infectious synthetic lipoparticle comprising: a small unilamellar vesicle comprising

- between 40 and 85 mol percent of a phosphatidylcholine as neutral amphiphile,
- at least one anionic lipid, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering.

The synthetic lipoparticles of the present invention comprise a Spike protein ectodomain that is bound to a lipid. The lipid is aggregated in the lipid bilayer such that the Spike protein ectodomain is arranged at the outside of the lipid bilayer. As the lipid bilayer forms a spherical chamber or vesicle, the Spike protein ectodomain is located at the outer surface of the bilayer so that the Spike protein can interact with cell surface receptors.

In other words, the present invention is directed to a non-infectious synthetic lipoparticle comprising: a vesicle-forming lipid bilayer comprising

- between 40 and 85 mol percent of a phosphatidylcholine as neutral amphiphile,
- at least one anionic lipid, and
- at least 1 mol percent of a lipid to which a Spike protein ectodomain is bound,

   wherein the Spike protein ectodomain binds to a cell surface receptor,
   wherein the lipoparticle has a diameter between 20 nm and 300 nm as measured by dynamic light scattering.

Reworded, the present invention is directed to a non-infectious synthetic lipoparticle comprising: a vesicle-forming lipid bilayer comprising

- between 40 and 85 mol percent of a phosphatidylcholine as neutral amphiphile,
- at least one anionic lipid, and
- at least 1 mol percent of a lipid to which a Spike protein ectodomain is bound,

   wherein the Spike protein ectodomain is positioned at the surface of the lipid bilayer vesicle and wherein the Spike protein ectodomain binds to a cell surface receptor,
   wherein the lipoparticle has a diameter between 20 nm and 300 nm as measured by dynamic light scattering.

## Description of the invention

The present invention is directed to synthetic lipoparticles comprising a lipid bilayer decorated with cell receptor binding proteins, wherein the lipid bilayer is molecularly defined to precisely mimic the biophysical features of various natural pathogens, such as viruses or virions.

Therefore, the present invention is directed to a non-infectious synthetic lipoparticle comprising: a lipid bilayer comprising

- between 40 and 85 mol percent of a phosphatidylcholine as neutral amphiphile,
- at least one anionic lipid, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering.

Suitable phosphatidylcholines for the lipid bilayer are exemplarily, but not restricted to, 1,2-dimyristoyl-sn-glycero-3-phosphocholine (DMPC), 1,2-dihexanoyl-sn-glycero-3-phosphocholine (DHPC), 1,2-dipalmitoleoyl-sn-glycero-3-phosphocholine, 1,2-dipalmitoyl-sn-glycero-3-phosphocholine (DPPC), 1-palmitoyl-2-oleoyl-glycero-3-phosphocholine (POPC), 1,2-distearoyl-sn-glycero-3-phosphocholine (DSPC), 1,2-dilauroyl-sn-glycero-3-phosphocholine (DLPC), and 1,2-dioleoyl-sn-glycero-3-phosphocholine (DOPC).

Suitable anionic lipids for the lipid bilayer are for example, but not limited to, phosphatidylserines, such as 1,2-dioleoyl-sn-glycero-3-phospho-l-serine (DOPS) and phosphatidylglycerols, such as 1,2-dioleoyl-sn-glycero-3-phospho-(1'-rac-glycerol) (DOPG), and phosphatidylinositols, such as 1,2-dioleoyl-sn-glycero-3-phospho-(1'-myo-inositol) (DOPI).

In one embodiment of the present invention, the non-infectious synthetic lipoparticle comprises a lipid bilayer comprising

- between 40 and 85 mol percent of a phosphatidylcholine as neutral amphiphile,
- a phosphatidylserine, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering.

In one embodiment of the present invention, the non-infectious synthetic lipoparticle comprises a lipid bilayer comprising

- between 40 and 85 mol percent of a phosphatidylcholine as neutral amphiphile,
- a phosphatidylglycerol, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering.

In one embodiment of the present invention, the non-infectious synthetic lipoparticle comprises a lipid bilayer comprising

- between 40 and 85 mol percent of a phosphatidylcholine as neutral amphiphile,
- a phosphatidylserine,
- a phosphatidylglycerol, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering.

In one embodiment of the present invention, the non-infectious synthetic lipoparticle comprises a lipid bilayer comprising

- between 40 and 85 mol percent of a phosphatidylcholine as neutral amphiphile,
- a phosphatidylserine,
- a phosphatidylinositol, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering.

In one embodiment of the present invention, the non-infectious synthetic lipoparticle comprises a lipid bilayer comprising

- between 40 and 85 mol percent of DOPC,
- DOPS, and

- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering.

In one embodiment of the present invention, the non-infectious synthetic lipoparticle comprises a lipid bilayer comprising

- between 40 and 85 mol percent of a phosphatidylcholine
- a phosphatidylethanolamine,
- a phosphatidylserine or a phosphatidylglycerol or a phosphatidylinositol, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering.
Preferably, the phosphatidylethanolamine is 1,2-dioleoyl-sn-glycero-3-phosphoethanolamine (DOPE)

In preferred embodiments, the lipid bilayer of the inventive lipoparticles is composed of at least one neutral amphiphile, at least one cationic lipid and at least one anionic lipid in order to optimally reproduce the biophysical properties of viral pathogens.

Thus, in one embodiment, the non-infectious synthetic lipoparticle comprises: a lipid bilayer comprising

- between 40 and 85 mol percent of a phosphatidylcholine as neutral amphiphile,
- at least one anionic lipid,
- at least one cationic lipid, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering.

Suitable cationic lipids for the lipid bilayer are for example, but not limited to, cholesterols, such as 18:1 cholesterol, 3β-[N-(N',N'-Dimethylaminoethane)-carbamoyl]cholesterol (DAC-Chol) and 3β-[N-(N',N'-dimethylaminoethane)-carbamoyl]cholesterol (DC-Chol), and sphingosylphosphorylcholine and N-acyl derivatives, such as N-palmitoyl-D-erythro-sphingosylphosphorylcholine, N-heptadecanoyl-D-erythro-sphingosylphosphorylcholine, N-oleoyl-D-erythro-sphingosylphosphorylcholine N-stearoyl-D-erythro-sphingosylphosphorylcholine, N-lignoceroyl-D-erythro-sphingosylphosphorylcholine, N-palmitoleoyl-D-erythrosphingosylphosphorylcholine.

In one embodiment of the present invention, the non-infectious synthetic lipoparticle comprises a lipid bilayer comprising

- between 40 and 85 mol percent of a phosphatidylcholine,
- a phosphatidylserine,
- a cholesterol, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering.

In one embodiment of the present invention, the non-infectious synthetic lipoparticle comprises a lipid bilayer comprising

- between 40 and 85 mol percent of a phosphatidylcholine
- a phosphatidylinositol
- a cholesterol, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering.

In one embodiment of the present invention, the non-infectious synthetic lipoparticle comprises a lipid bilayer comprising

- between 40 and 85 mol percent of a phosphatidylcholine,
- a phosphatidylinositol,

- a cholesterol,
- a sphingosylphosphorylcholine, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering.

In one embodiment of the present invention, the non-infectious synthetic lipoparticle comprises a lipid bilayer comprising

- between 40 and 85 mol percent of a phosphatidylcholine
- a phosphatidylethanolamine
- a phosphatidylinositol
- a cholesterol
- a sphingosylphosphorylcholine, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering.

In another embodiment, the non-infectious synthetic lipoparticle comprises: a lipid bilayer comprising

- 40-50 mol% 1,2-dioleoyl-sn-glycero-3-phosphocholine,
- 15-25 mol% 1,2-dioleoyl-sn-glycero-3-phosphoethanolamine,
- 2-4 mol% 1,2-dioleoyl-sn-glycero-3-phospho-l-serine,
- 12 mol% 1,2-dioleoyl-sn-glycero-3-phospho-(1'-myo-inositol),
- 14 mol% cholesterol,
- 3 mol% N-stearoyl-D-erythro-sphingosylphosphorylcholine, and
- 1 mol% of a lipid-bound Spike protein ectodomain, wherein the Spike protein ectodomain binds to a cell surface receptor,

wherein the lipoparticle has a diameter between 20 nm and 300 nm as measured by dynamic light scattering.

In another embodiment, the non-infectious synthetic lipoparticle comprises: a lipid bilayer comprising

- 80-85 mol% 1,2-dioleoyl-sn-glycero-3-phosphocholine,
- 5-10 mol% 1,2-dioleoyl-sn-glycero-3-phosphoethanolamine,
- 5 mol% 1,2-dioleoyl-sn-glycero-3-phospho-(1'-myo-inositol),
- 3 mol% N-stearoyl-D-erythro-sphingosylphosphorylcholine, and
- 1 mol% of a lipid-bound Spike protein ectodomain, wherein the Spike protein ectodomain binds to a cell surface receptor,

wherein the lipoparticle has a diameter between 20 nm and 300 nm as measured by dynamic light scattering.

In preferred embodiments, the lipid bilayer of the inventive lipoparticles may further comprise a neutral lipid, preferably a ceramide, such as N-stearoyl-D-erythro-sphingosine.

In one embodiment of the present invention, the non-infectious synthetic lipoparticle comprises a lipid bilayer comprising

- between 40 and 85 mol percent of a phosphatidylcholine,
- a phosphatidylserine,
- a cholesterol,
- a ceramide, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering.

In one embodiment of the present invention, the non-infectious synthetic lipoparticle comprises a lipid bilayer comprising

- between 40 and 85 mol percent of a phosphatidylcholine,
- a phosphatidylinositol,
- a cholesterol, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering.

In one embodiment of the present invention, the non-infectious synthetic lipoparticle comprises a lipid bilayer comprising

- between 40 and 85 mol percent of a phosphatidylcholine,
- a phosphatidylinositol,
- a cholesterol,
- a sphingosylphosphorylcholine,
- a ceramide, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering.

In one embodiment of the present invention, the non-infectious synthetic lipoparticle comprises a lipid bilayer comprising

- between 40 and 85 mol percent of a phosphatidylcholine,
- a phosphatidylethanolamines,
- a phosphatidylinositol,
- a cholesterol,
- a sphingosylphosphorylcholine,
- a ceramide, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering.

In a preferred embodiment, the non-infectious synthetic lipoparticle comprises: a lipid bilayer comprising

| - 40 | mol% | 1,2-dioleoyl-sn-glycero-3-phosphocholine, |
| - 30 | mol% | 1,2-dioleoyl-sn-glycero-3-phosphoethanolamine, |
| - 22 | mol% | *N*-stearoyl-D-erythro-sphingosylphosphorylcholine, |
| - 5 | mol% | N-stearoyl-D-erythro-sphingosine, and |
| - 1 | mol% | of a lipid-bound Spike protein ectodomain, wherein the Spike protein ectodomain binds to a cell surface receptor, |

wherein the lipoparticle has a diameter between 20 nm and 300 nm as measured by dynamic light scattering.

The Spike protein ectodomain can be bound to the lipid by any conventional binding method such as covalent binding (ester bond formation or amide bond formation) or affinity binding (e.g. $Ni^{2+}$/His-tag). To this extent, the lipid is equipped with suitable reactive groups. In other words, the lipid is a reactive lipid in such a manner that it can bind or immobilise the Spike protein ectodomain. Correspondingly, the Spike protein ectodomain is also equipped with a reactive group or a tag for binding or immobilising to the lipid. The Spike protein ectodomain can be bound to the lipid prior vesicle formation or the Spike protein ectodomain can be bound to the already formed lipid bilayer vesicle. The latter approach has the advantage that all Spike protein ectodomain is located at the outer surface of the lipid bilayer vesicle and none of it in the inner cavity of the vesicle, where it cannot interact with a cell surface receptor.

Thus, the present invention is directed to a non-infectious synthetic lipoparticle comprising: a vesicle-forming lipid bilayer comprising

- between 40 and 85 mol percent of a phosphatidylcholine as neutral amphiphile,
- at least one anionic lipid, and
- at least 1 mol percent of a reactive lipid,

wherein a Spike protein ectodomain is bound to the reactive lipid of the lipid bilayer vesicle and wherein the Spike protein ectodomain binds to a cell surface receptor,
wherein the lipoparticle has a diameter between 20 nm and 300 nm as measured by dynamic light scattering.

The Spike protein ectodomain can be any protein which interacts or binds to a cell surface receptor, preferably a receptor of a human cell. Thus, the present invention is also directed to a non-infectious synthetic lipoparticle comprising: a lipid bilayer comprising

- between 40 and 85 mol percent of a phosphatidylcholine as neutral amphiphile,
- at least one anionic lipid, and
- at least 1 mol percent of a lipid-bound Spike protein ectodomain, wherein the Spike protein ectodomain binds to a human cell surface receptor,

wherein the lipoparticle has a diameter between 20 nm and 300 nm as measured by dynamic light scattering.

In one embodiment, the Spike protein ectodomain is bound via a His-tag to a Ni-NTA functionalized lipid. Thus, the present invention is directed to a non-infectious synthetic lipoparticle comprising: a vesicle-forming lipid bilayer comprising

- between 40 and 85 mol percent of a phosphatidylcholine as neutral amphiphile,
- at least one anionic lipid, and
- at least 1 mol percent of a lipid-bound Spike protein ectodomain, wherein the Spike protein ectodomain is bound via a His-tag to a Ni-NTA functionalized lipid and wherein the Spike protein ectodomain binds to a cell surface receptor,

wherein the lipoparticle has a diameter between 20 nm and 300 nm as measured by dynamic light scattering. Preferably, the Ni-NTA functionalized lipid is the nickel salt of 1,2-dioleoyl-sn-glycero-3-[(N-(5-amino-1-carboxypentyl)iminodiacetic acid)succinyl].

The Spike protein ectodomain may be derived from any surface protein which is known for binding to cell surface receptors and/or which is known for being involved in viral entry mechanism of viruses.
Thus, the present invention is also directed to a non-infectious synthetic lipoparticle comprising: a lipid bilayer comprising

- between 40 and 85 mol percent of a phosphatidylcholine as neutral amphiphile,
- at least one anionic lipid, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering, and wherein the Spike protein ectodomain is selected from the SARS-CoV-2 Spike protein ectodomain or a mutant thereof, SARS-CoV Spike protein ectodomain or a mutant thereof, MERS-CoV Spike protein ectodomain or a mutant thereof, human immunodeficiency virus type 1 envelope Spike protein ectodomain or a mutant thereof, large Hepatitis B virus envelope Spike protein ectodomain or a mutant thereof, Hepatitis C virus or a mutant thereof, Herpes simplex virus 1 glycoprotein B, C, D, H, or L ectodomain or a mutant thereof, Herpes simplex virus 2 glycoprotein B, C, D, H, or L ectodomain or a mutant thereof, Ebolavirus glycoprotein ectodomain or a mutant thereof, Marburg virus glycoprotein ectodomain or a mutant thereof, Influenza virus A hemagglutinin ectodomain or mutant thereof, Influenza virus B hemagglutinin ectodomain or mutant thereof, Influenza virus C hemagglutinin ectodomain or mutant thereof, Influenza virus D hemagglutinin ectodomain or mutant thereof, Dengue virus E envelope protein ectodomain or a mutant thereof, Zika virus Spike protein ectodomain or a mutant thereof, a mutant thereof.

In a preferred embodiment of the inventive lipoparticle, the Spike protein ectodomain is selected from SARS-CoV-2 Spike protein ectodomain or a mutant thereof, SARS-CoV Spike protein ectodomain or a mutant thereof, MERS-CoV Spike protein ectodomain or a mutant thereof, HIV-1 gp 120 protein, Hepatitis B Surface Antigen adw subtype 2 (HBVsAG), Hepatitis C E1 protein, Ebolavirus glycoprotein, Marburg virus glycoprotein (AMARV GP), Influenza virus A hemagglutinin H1 protein, and Dengue virus 2 E-glycoprotein.

Thus, the present invention is also directed to a non-infectious synthetic lipoparticle comprising: a lipid bilayer comprising

- between 40 and 85 mol percent of a phosphatidylcholine as neutral amphiphile,
- at least one anionic lipid, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering, and wherein the Spike protein ectodomain is selected from SARS-CoV-2 Spike protein ectodomain or a mutant thereof, SARS-CoV Spike protein ectodomain or a mutant thereof, MERS-CoV Spike protein ectodomain or a mutant thereof, HIV-1 gp 120 protein, Hepatitis B Surface Antigen adw subtype 2 (HBVsAG), Hepatitis C E1 protein, Ebolavirus glycoprotein, Marburg virus glycoprotein (AMARV GP), Influenza virus A hemagglutinin H1 protein, and Dengue virus 2 E-glycoprotein.

In a further preferred embodiment of the inventive lipoparticle, the Spike protein ectodomain is a SARS-CoV-2 Spike protein ectodomain mutant homotrimer. Thus, the present invention is also directed to a non-infectious synthetic lipoparticle comprising: a lipid bilayer comprising

- between 40 and 85 mol percent of a phosphatidylcholine as neutral amphiphile,
- at least one anionic lipid, and
- at least 1 mol percent of a lipid-bound SARS-CoV-2 Spike protein ectodomain mutant homotrimer, wherein the homotrimer binds to a cell surface receptor,

wherein the lipoparticle has a diameter between 20 nm and 300 nm as measured by dynamic light scattering.

In a preferred embodiment of the inventive lipoparticle, the Spike protein ectodomain is a SARS-CoV-2 Spike protein ectodomain mutant homotrimer, wherein each monomer of the SARS-CoV-2 Spike protein ectodomain comprises an amino acid sequence identical to the sequence as set forth in SEQ ID NO 5 and wherein each monomer comprises further a single point mutation at one of the following sites: L4, T6, P12, D66, D124, R176, D201, R232, K403, L411, E470, N487, A556, D600, H641, Q663, P667, A684, T699, S965, T1010, and D1101. Thus, the present invention is also directed to a non-infectious synthetic lipoparticle comprising: a lipid bilayer comprising

- between 40 and 85 mol percent of a phosphatidylcholine as neutral amphiphile,
- at least one anionic lipid, and
- at least 1 mol percent of a lipid-bound SARS-CoV-2 Spike protein ectodomain mutant homotrimer, wherein the homotrimer binds to a cell surface receptor,

wherein the lipoparticle has a diameter between 20 nm and 300 nm as measured by dynamic light scattering, and wherein each monomer of the SARS-CoV-2 Spike protein ectodomain comprises an amino acid sequence identical to the sequence as set forth in SEQ ID NO 5 and wherein each monomer comprises further a single point mutation at one of the following sites: L4, T6, P12, D66, D124, R176, D201, R232, K403, L411, E470, N487, A556, D600, H641, Q663, P667, A684, T699, S965, T1010, and D1101.

In a preferred embodiment of the inventive lipoparticle, the Spike protein ectodomain is a SARS-CoV-2 Spike protein ectodomain mutant homotrimer, wherein each monomer comprises an amino acid sequence identical to one of the sequences as set forth in SEQ ID NOs 6 to 29. Thus, the present invention is also directed to a non-infectious synthetic lipoparticle comprising: a lipid bilayer comprising

- between 40 and 85 mol percent of a phosphatidylcholine as neutral amphiphile,
- at least one anionic lipid, and
- at least 1 mol percent of a lipid-bound SARS-CoV-2 Spike protein ectodomain mutant homotrimer, wherein the homotrimer binds to a cell surface receptor,

wherein the lipoparticle has a diameter between 20 nm and 300 nm as measured by dynamic light scattering, and wherein each monomer of the SARS-CoV-2 Spike protein ectodomain mutant homotrimer comprises an amino acid sequence identical to one of the sequences as set forth in SEQ ID NOs 6 to 29.

Spike protein ectodomains usually contain free fatty acids when they are produced by recombinant techniques. The inventors have found that these free fatty acids (e.g. palmitic acid, oleic acid, linoleic acid and arachidonic acid) significantly influence the receptor binding of the Spike protein ectodomains. For receptor binding studies it is therefore essential to control the free fatty acids content. Removal of free fatty acids can be achieved by treating the proteins with Lipidex-1000 (a hydroxypropyl beaded dextran substituted with approximately 10% by weight long chain alkyl ethers averaging 15 carbons in length), thereby leading to the corresponding apo Spike protein ectodomains. Thus, the present invention is also directed to a non-infectious synthetic lipoparticle comprising: a lipid bilayer comprising

- between 40 and 85 mol percent of a phosphatidylcholine as neutral amphiphile,
- at least one anionic lipid, and
- at least 1 mol percent of a lipid-bound apo Spike protein ectodomain, wherein the Spike protein ectodomain binds to a cell surface receptor and is devoid of free fatty acids,

wherein the lipoparticle has a diameter between 20 nm and 300 nm as measured by dynamic light scattering.

The inventive lipoparticle can have a DLS diameter between 20 nm and 300 nm to optimally reproduce the biophysical properties of viral pathogens. Suitable DLS diameters are between 20 nm and 300 nm, and more preferably between 80 nm and 140 nm. Preferably, the diameter of the inventive lipoparticle is between 100 nm and 120 nm. Thus, in one embodiment, the non-infectious synthetic lipoparticle comprises: a lipid bilayer comprising

- between 40 and 85 mol percent of a phosphatidylcholine as neutral amphiphile,
- at least one anionic lipid, and
- at least 1 mol percent of a lipid-bound Spike protein ectodomain, wherein the Spike protein ectodomain binds to a cell surface receptor,

wherein the lipoparticle has a diameter between 80 nm and 140 nm as measured by dynamic light scattering.

In a further embodiment, the non-infectious synthetic lipoparticle comprises: a lipid bilayer comprising

- between 40 and 85 mol percent of a phosphatidylcholine as neutral amphiphile,
- at least one anionic lipid, and
- at least 1 mol percent of a lipid-bound Spike protein ectodomain, wherein the Spike protein ectodomain binds to a cell surface receptor,

wherein the lipoparticle has a diameter between 100 nm and 120 nm as measured by dynamic light scattering.

In a further embodiment, the non-infectious synthetic lipoparticle comprises: a lipid bilayer comprising

- between 40 and 85 mol percent of a phosphatidylcholine as neutral amphiphile,
- at least one anionic lipid, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering, wherein the cell surface receptor is selected from angiotensin-converting enzyme 2, DPP4, CD4, CCR5, NTCP, CD81, SR-BI, DC-SIGN, L-SIGN, heparan sulphate proteoglycans, asialoglycoprotein receptors, herpesvirus entry mediator, nectin-1, nectin-2, Sialic acid-containing receptors, $nLc_4Cer$, HSP70/HSP90, GRP78, Laminin receptor and TIM-1. Preferably, the cell surface receptor is angiotensin-converting enzyme 2 (ACE2).

For studying receptor binding, the lipoparticle may further comprise a **fluorophore** in the lipid bilayer. The fluorophore may be immobilized to the surface of the lipid bilayer by a bond to a lipid which is aggregated in the lipid bilayer. Thus, in a further embodiment, the non-infectious synthetic lipoparticle comprises: a lipid bilayer comprising

- between 40 and 85 mol percent of a phosphatidylcholine as neutral amphiphile,
- at least one anionic lipid,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering.

Reworded, the non-infectious synthetic lipoparticle comprises: a lipid bilayer comprising

- between 40 and 85 mol percent of a phosphatidylcholine as neutral amphiphile,
- at least one anionic lipid,
- a lipid fluorophore conjugate, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering.

Preferably, the fluorophore is bound or conjugated to a neutral amphiphile as lipid, such as a phosphatidylethanolamine. Preferably, the fluorophore is bound or conjugated to 1,2-dioleoyl-sn-glycero-3-phosphoethanolamine (DOPE).

Thus, in one embodiment, the non-infectious synthetic lipoparticle comprises: a lipid bilayer comprising

- between 40 and 85 mol percent of a phosphatidylcholine as neutral amphiphile,

- at least one anionic lipid,
- a fluorophore phosphatidylethanolamine conjugate, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering.

The fluorophore may be any suitable molecular probe, which has an absorption wavelength above 500 nm, such as Lissamine Rhodamine B, fluorescein, and Atto488 (CAS number 923585-42-6).

Preferably, the lipid-bound fluorophore may be present in the lipid bilayer of the inventive lipoparticles in the amount of 0.1 mol% to 5 mol%, more preferably 0.2 mol% to 4 mol%, more preferably 0.3 mol% to 3.5 mol%, more preferably 0.4 mol% to 3 mol%, more preferably 0.5 mol% to 2.5 mol%, more preferably 0.6 mol% to 2 mol%, more preferably 0.7 mol% to 1.5 mol%, and most preferably 0.8 mol% to 1.2 mol%. Preferably, the lipid-bound fluorophore is present in the lipid bilayer of the inventive lipoparticles in the amount of 1 mol%.

Thus, in a further embodiment, the non-infectious synthetic lipoparticle comprises: a lipid bilayer comprising

- between 40 and 85 mol percent of a phosphatidylcholine as neutral amphiphile,
- at least one anionic lipid,
- 1 mol percent of a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering.

In one embodiment, the non-infectious synthetic lipoparticle comprises: a lipid bilayer comprising

- between 40 and 85 mol percent of a phosphatidylcholine as neutral amphiphile,
- at least one anionic lipid,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 80 nm and 140 nm as measured by dynamic light scattering.

In one embodiment, the non-infectious synthetic lipoparticle comprises: a lipid bilayer comprising

- between 40 and 85 mol percent of a phosphatidylcholine as neutral amphiphile,
- at least one anionic lipid,
- 1 mol percent of a lipid-bound fluorophore, and
- at least 1 mol percent of a lipid-bound Spike protein ectodomain, wherein the Spike protein ectodomain binds to a cell surface receptor,

wherein the lipoparticle has a diameter between 80 nm and 140 nm as measured by dynamic light scattering.

In one embodiment of the present invention, the non-infectious synthetic lipoparticle comprises a lipid bilayer comprising

- between 40 and 85 mol percent of a phosphatidylcholine as neutral amphiphile,
- a phosphatidylserine,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering.

In one embodiment of the present invention, the non-infectious synthetic lipoparticle comprises a lipid bilayer comprising

- between 40 and 85 mol percent of a phosphatidylcholine as neutral amphiphile,
- a phosphatidylglycerol,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering.

In one embodiment of the present invention, the non-infectious synthetic lipoparticle comprises a lipid bilayer comprising

- between 40 and 85 mol percent of a phosphatidylcholine as neutral amphiphile,
- a phosphatidylserine,
- a phosphatidylglycerol,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering.

In one embodiment of the present invention, the non-infectious synthetic lipoparticle comprises a lipid bilayer comprising

- between 40 and 85 mol percent of a phosphatidylcholine as neutral amphiphile,
- a phosphatidylserine,
- a phosphatidylinositol,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering.

In one embodiment of the present invention, the non-infectious synthetic lipoparticle comprises a lipid bilayer comprising

- between 40 and 85 mol percent of DOPC,
- DOPS,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering.

In one embodiment of the present invention, the non-infectious synthetic lipoparticle comprises a lipid bilayer comprising

- between 40 and 85 mol percent of a phosphatidylcholine
- a phosphatidylethanolamine,
- a phosphatidylserine or a phosphatidylglycerol or a phosphatidylinositol,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering. Preferably, the phosphatidylethanolamine is 1,2-dioleoyl-sn-glycero-3-phosphoethanolamine (DOPE).

In a further embodiment, the non-infectious synthetic lipoparticle comprises: a lipid bilayer comprising

- between 40 and 85 mol percent of a phosphatidylcholine as neutral amphiphile,
- at least one anionic lipid,
- at least one cationic lipid,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering.

In one embodiment of the present invention, the non-infectious synthetic lipoparticle comprises a lipid bilayer comprising

- between 40 and 85 mol percent of a phosphatidylcholine
- a phosphatidylserine
- a cholesterol,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering.

In one embodiment of the present invention, the non-infectious synthetic lipoparticle comprises a lipid bilayer comprising

- between 40 and 85 mol percent of a phosphatidylcholine
- a phosphatidylinositol
- a cholesterol,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering.

In one embodiment of the present invention, the non-infectious synthetic lipoparticle comprises a lipid bilayer comprising

- between 40 and 85 mol percent of a phosphatidylcholine
- a phosphatidylinositol
- a cholesterol
- a sphingosylphosphorylcholine,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering.

In one embodiment of the present invention, the non-infectious synthetic lipoparticle comprises a lipid bilayer comprising

- between 40 and 85 mol percent of a phosphatidylcholine
- a phosphatidylethanolamine
- a phosphatidylinositol
- a cholesterol
- a sphingosylphosphorylcholine,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering.

In another embodiment, the non-infectious synthetic lipoparticle comprises: a lipid bilayer comprising

| | | |
|---|---|---|
| - 40-50 | mol% | 1,2-dioleoyl-sn-glycero-3-phosphocholine, |
| - 15-25 | mol% | 1,2-dioleoyl-sn-glycero-3-phosphoethanolamine, |
| - 2-4 | mol% | 1,2-dioleoyl-sn-glycero-3-phospho-l-serine, |
| - 12 | mol% | 1,2-dioleoyl-sn-glycero-3-phospho-(1'-myo-inositol), |
| - 14 | mol% | cholesterol, |
| - 3 | mol% | *N*-stearoyl-D-erythro-sphingosylphosphorylcholine, |
| - 1 | mol% | 1,2-dioleoyl-sn-glycero-3-phosphoethanolamine-*N*-(lissamine rhodamine B Sulfonyl), and |
| - 1 | mol% | of a lipid-bound Spike protein ectodomain, wherein the Spike protein ectodomain binds to a cell surface receptor, |

wherein the lipoparticle has a diameter between 20 nm and 300 nm as measured by dynamic light scattering. Preferably, the Spike protein ectodomain is bound via a His-Tag to Ni-NTA functionalized lipid, preferably the nickel salt of 1,2-dioleoyl-sn-glycero-3-[(N-(5-amino-1-carboxypentyl)iminodiacetic acid)succinyl].

In another embodiment, the non-infectious synthetic lipoparticle comprises: a lipid bilayer comprising

| - 80-85 | mol% | 1,2-dioleoyl-sn-glycero-3-phosphocholine, |
| - 5-10 | mol% | 1,2-dioleoyl-sn-glycero-3-phosphoethanolamine, |
| - 5 | mol% | 1,2-dioleoyl-sn-glycero-3-phospho-(1 '-myo-inositol), |
| - 3 | mol% | *N*-stearoyl-D-erythro-sphingosylphosphorylcholine, |
| - 1 | mol% | 1,2-dioleoyl-sn-glycero-3-phosphoethanolamine-*N*-(lissamine rhodamine B Sulfonyl), and |
| - 1 | mol% | of a lipid-bound Spike protein ectodomain, wherein the Spike protein ectodomain binds to a cell surface receptor, |

wherein the lipoparticle has a diameter between 20 nm and 300 nm as measured by dynamic light scattering. Preferably, the Spike protein ectodomain is bound via a His-Tag to Ni-NTA functionalized lipid, preferably the nickel salt of 1,2-dioleoyl-sn-glycero-3-[(N-(5-amino-1-carboxypentyl)iminodiacetic acid)succinyl].

In preferred embodiments, the lipid bilayer of the inventive lipoparticles may further comprise a neutral lipid, preferably a ceramide, such as N-stearoyl-D-erythro-sphingosine.

In one embodiment of the present invention, the non-infectious synthetic lipoparticle comprises a lipid bilayer comprising

- between 40 and 85 mol percent of a phosphatidylcholine,
- a phosphatidylserine,
- a cholesterol,
- a ceramide,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering.

In one embodiment of the present invention, the non-infectious synthetic lipoparticle comprises a lipid bilayer comprising

- between 40 and 85 mol percent of a phosphatidylcholine,
- a phosphatidylinositol,
- a cholesterol,
- a sphingosylphosphorylcholine,
- a ceramide,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering.

In one embodiment of the present invention, the non-infectious synthetic lipoparticle comprises a lipid bilayer comprising

- between 40 and 85 mol percent of a phosphatidylcholine
- a phosphatidylethanolamine
- a phosphatidylinositol
- a cholesterol
- a sphingosylphosphorylcholine
- a ceramide,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering.

In a preferred embodiment, the non-infectious synthetic lipoparticle comprises: a lipid bilayer comprising

| | | |
|---|---|---|
| - 40 | mol% | 1,2-dioleoyl-sn-glycero-3-phosphocholine, |
| - 30 | mol% | 1,2-dioleoyl-sn-glycero-3-phosphoethanolamine, |
| - 22 | mol% | N-stearoyl-D-erythro-sphingosylphosphorylcholine, |
| - 5 | mol% | N-stearoyl-D-erythro-sphingosine, |
| - 1 | mol% | 1,2-dioleoyl-sn-glycero-3-phosphoethanolamine-N-(lissamine rhodamine B Sulfonyl), and |
| - 1 | mol% | of a lipid-bound Spike protein ectodomain, wherein the Spike protein ectodomain binds to a cell surface receptor, |

wherein the lipoparticle has a diameter between 20 nm and 300 nm as measured by dynamic light scattering. Preferably, the Spike protein ectodomain is bound via a His-Tag to Ni-NTA functionalized lipid, preferably the nickel salt of 1,2-dioleoyl-sn-glycero-3-[(N-(5-amino-1-carboxypentyl)iminodiacetic acid)succinyl].

In one embodiment, the non-infectious synthetic lipoparticle comprises: a lipid bilayer comprising

- between 40 and 85 mol percent of a phosphatidylcholine as neutral amphiphile,
- at least one anionic lipid,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering, and wherein the Spike protein ectodomain is selected from the SARS-CoV-2 Spike protein ectodomain or a mutant thereof, SARS-CoV Spike protein ectodomain or a mutant thereof, MERS-CoV Spike protein ectodomain or a mutant thereof, human immunodeficiency virus type 1 envelope Spike protein ectodomain or a mutant thereof, large Hepatitis B virus envelope Spike protein ectodomain or a mutant thereof, Hepatitis C virus or a mutant thereof, Herpes simplex virus 1 glycoprotein B, C, D, H, or L ectodomain or a mutant thereof, Herpes simplex virus 2 glycoprotein B, C, D, H, or L ectodomain or a mutant thereof, Ebolavirus glycoprotein ectodomain or a mutant thereof, Marburg virus glycoprotein ectodomain or a mutant thereof, Influenza virus A hemagglutinin ectodomain or mutant thereof, Influenza virus B hemagglutinin ectodomain or mutant thereof, Influenza virus C hemagglutinin ectodomain or mutant thereof, Influenza virus D hemagglutinin ectodomain or mutant thereof, Dengue virus E envelope protein ectodomain or a mutant thereof, Zika virus Spike protein ectodomain or a mutant thereof, a mutant thereof; preferably selected from SARS-CoV-2 Spike protein ectodomain or a mutant thereof, SARS-CoV Spike protein ectodomain or a mutant thereof, MERS-CoV Spike protein ectodomain or a mutant thereof, HIV-1 gp 120 protein, Hepatitis B Surface Antigen adw subtype 2 (HBVsAG), Hepatitis C E1 protein, Ebolavirus glycoprotein, Marburg virus glycoprotein (AMARV GP), Influenza virus A hemagglutinin H1 protein, and Dengue virus 2 E-glycoprotein.

In a preferred embodiment, the non-infectious synthetic lipoparticle comprises: a lipid bilayer comprising

- between 40 and 85 mol percent of a phosphatidylcholine as neutral amphiphile,
- at least one anionic lipid,
- a lipid-bound fluorophore, and
- at least 1 mol percent of a lipid-bound SARS-CoV-2 Spike protein ectodomain mutant homotrimer, wherein the homotrimer binds to a cell surface receptor,

wherein the lipoparticle has a diameter between 20 nm and 300 nm as measured by dynamic light scattering. Preferably, each monomer of the SARS-CoV-2 Spike protein ectodomain comprises an amino acid sequence identical to the sequence as set forth in SEQ ID NO 5 and wherein each monomer comprises further a single point mutation at one of the following sites: L4, T6, P12, D66, D124, R176, D201, R232, K403, L411, E470, N487, A556, D600, H641, Q663, P667, A684, T699, S965, T1010, and D1101. More preferably, each monomer of the SARS-CoV-2 Spike protein ectodomain mutant homotrimer comprises an amino acid sequence identical to one of the sequences as set forth in SEQ ID NOs 6 to 29.

In one embodiment, the non-infectious synthetic lipoparticle comprises: a lipid bilayer comprising

- between 40 and 85 mol percent of a phosphatidylcholine as neutral amphiphile,
- at least one anionic lipid,
- a lipid-bound fluorophore, and
- at least 1 mol percent of a lipid-bound Spike protein ectodomain, wherein the Spike protein ectodomain binds to a cell surface receptor and is devoid of free fatty acids,

wherein the lipoparticle has a diameter between 20 nm and 300 nm as measured by dynamic light scattering.

In one embodiment, the non-infectious synthetic lipoparticle comprises: a lipid bilayer comprising

- between 40 and 85 mol percent of a phosphatidylcholine as neutral amphiphile,
- at least one anionic lipid,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering, and wherein the cell surface receptor is selected from angiotensin-converting enzyme 2, DPP4, CD4, CCR5, NTCP, CD81, SR-BI, DC-SIGN, L-SIGN, heparan sulphate proteoglycans, asialoglycoprotein receptors, herpesvirus entry mediator, nectin-1, nectin-2, Sialic acid-containing receptors, $nLc_4Cer$, HSP70/HSP90, GRP78, Laminin receptor and TIM-1. Preferably, the cell surface receptor is angiotensin-converting enzyme 2 (ACE2).

A further aspect of the present invention is directed to a **method for quantification of Spike mediated binding** of a lipoparticle to a cell in a sample comprising the steps:

(a) providing a lipoparticle comprising: a lipid bilayer comprising

- between 40 and 85 mol percent of a phosphatidylcholine as neutral amphiphile,
- at least one anionic lipid,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering;
(b) contacting the lipoparticle with a sample, to bind the lipoparticle to the cells contained in the sample;
(c) washing the sample, thereby removing the lipoparticles not bound to the cells;
(d) detecting or determining fluorescence in the washed sample, thereby detecting or determining the number of lipoparticles bound to the cells in the sample.

In a preferred embodiment, the total number of lipoparticles in the sample is detected or determined prior to washing the sample. Thus, the inventive method for quantification of Spike mediated binding of the lipoparticle to a cell in a sample comprises the steps:

(a) providing a lipoparticle comprising: a lipid bilayer comprising

- between 40 and 85 mol percent of a phosphatidylcholine as neutral amphiphile,
- at least one anionic lipid,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering;
(b) contacting the lipoparticle with a sample, to bind the lipoparticle to the cells contained in the sample;
(c') detecting or determining fluorescence in the sample, thereby detecting or determining the total number of lipoparticles in the sample;
(c) washing the sample, thereby removing the lipoparticles not bound to the cells:
(d) detecting or determining fluorescence in the washed sample, thereby detecting or determining the number of lipoparticles bound to the cells in the sample.

In another embodiment, the inventive method for quantification of Spike mediated binding of the lipoparticle to a cell in a sample comprises the steps:

(a) providing a lipoparticle comprising: a lipid bilayer comprising

- between 40 and 85 mol percent of a phosphatidylcholine as neutral amphiphile,
- a phosphatidylserine,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering;
(b) contacting the lipoparticle with a sample, to bind the lipoparticle to the cells contained in the sample;
(c') detecting or determining fluorescence in the sample, thereby detecting or determining the total number of lipoparticles in the sample;
(c) washing the sample, thereby removing the lipoparticles not bound to the cells:
(d) detecting or determining fluorescence in the washed sample, thereby detecting or determining the number of lipoparticles bound to the cells in the sample.

In another embodiment, the inventive method for quantification of Spike mediated binding of the lipoparticle to a cell in a sample comprises the steps:

(a) providing a lipoparticle comprising: a lipid bilayer comprising

- between 40 and 85 mol percent of a phosphatidylcholine as neutral amphiphile,
- a phosphatidylglycerol,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering;
(b) contacting the lipoparticle with a sample, to bind the lipoparticle to the cells contained in the sample;
(c') detecting or determining fluorescence in the sample, thereby detecting or determining the total number of lipoparticles in the sample;
(c) washing the sample, thereby removing the lipoparticles not bound to the cells:
(d) detecting or determining fluorescence in the washed sample, thereby detecting or determining the number of lipoparticles bound to the cells in the sample.

In another embodiment, the inventive method for quantification of Spike mediated binding of the lipoparticle to a cell in a sample comprises the steps:

(a) providing a lipoparticle comprising: a lipid bilayer comprising

- between 40 and 85 mol percent of a phosphatidylcholine as neutral amphiphile,
- a phosphatidylserine,
- a phosphatidylglycerol,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering;
(b) contacting the lipoparticle with a sample, to bind the lipoparticle to the cells contained in the sample;
(c') detecting or determining fluorescence in the sample, thereby detecting or determining the total number of lipoparticles in the sample;
(c) washing the sample, thereby removing the lipoparticles not bound to the cells:
(d) detecting or determining fluorescence in the washed sample, thereby detecting or determining the number of lipoparticles bound to the cells in the sample.

In another embodiment, the inventive method for quantification of Spike mediated binding of the lipoparticle to a cell in a sample comprises the steps:

(a) providing a lipoparticle comprising: a lipid bilayer comprising

- between 40 and 85 mol percent of a phosphatidylcholine as neutral amphiphile,
- a phosphatidylserine,
- a phosphatidylinositol,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering;
(b) contacting the lipoparticle with a sample, to bind the lipoparticle to the cells contained in the sample;
(c') detecting or determining fluorescence in the sample, thereby detecting or determining the total number of lipoparticles in the sample;
(c) washing the sample, thereby removing the lipoparticles not bound to the cells:
(d) detecting or determining fluorescence in the washed sample, thereby detecting or determining the number of lipoparticles bound to the cells in the sample.

In another embodiment, the inventive method for quantification of Spike mediated binding of the lipoparticle to a cell in a sample comprises the steps:

(a) providing a lipoparticle comprising: a lipid bilayer comprising

- between 40 and 85 mol percent of DOPC,
- DOPS,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering;
(b) contacting the lipoparticle with a sample, to bind the lipoparticle to the cells contained in the sample;
(c') detecting or determining fluorescence in the sample, thereby detecting or determining the total number of lipoparticles in the sample;
(c) washing the sample, thereby removing the lipoparticles not bound to the cells:
(d) detecting or determining fluorescence in the washed sample, thereby detecting or determining the number of lipoparticles bound to the cells in the sample.

In another embodiment, the inventive method for quantification of Spike mediated binding of the lipoparticle to a cell in a sample comprises the steps:

(a) providing a lipoparticle comprising: a lipid bilayer comprising

- between 40 and 85 mol percent of a phosphatidylcholine,
- a phosphatidylethanolamine,
- a phosphatidylserine or a phosphatidylglycerol or a phosphatidylinositol,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering;
(b) contacting the lipoparticle with a sample, to bind the lipoparticle to the cells contained in the sample;
(c') detecting or determining fluorescence in the sample, thereby detecting or determining the total number of lipoparticles in the sample;
(c) washing the sample, thereby removing the lipoparticles not bound to the cells:
(d) detecting or determining fluorescence in the washed sample, thereby detecting or determining the number of lipoparticles bound to the cells in the sample.

Preferably, the phosphatidylethanolamine is 1,2-dioleoyl-sn-glycero-3-phosphoethanolamine (DOPE).
In another embodiment, the inventive method for quantification of Spike mediated binding of the lipoparticle to a cell in a sample comprises the steps:

(a) providing a lipoparticle comprising: a lipid bilayer comprising

- a phosphatidylethanolamine
- a phosphatidylinositol
- a cholesterol
- a sphingosylphosphorylcholine,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering;
(b) contacting the lipoparticle with a sample, to bind the lipoparticle to the cells contained in the sample;
(c') detecting or determining fluorescence in the sample, thereby detecting or determining the total number of lipoparticles in the sample;
(c) washing the sample, thereby removing the lipoparticles not bound to the cells:
(d) detecting or determining fluorescence in the washed sample, thereby detecting or determining the number of lipoparticles bound to the cells in the sample.

In another embodiment, the inventive method for quantification of Spike mediated binding of the lipoparticle to a cell in a sample comprises the steps:
(a) providing a lipoparticle comprising: a lipid bilayer comprising

| | | |
|---|---|---|
| - 40-50 | mol% | 1,2-dioleoyl-sn-glycero-3-phosphocholine, |
| - 15-25 | mol% | 1,2-dioleoyl-sn-glycero-3-phosphoethanolamine, |
| - 2-4 | mol% | 1,2-dioleoyl-sn-glycero-3-phospho-l-serine, |
| - 12 | mol% | 1,2-dioleoyl-sn-glycero-3-phospho-(1'-myo-inositol), |
| - 14 | mol% | cholesterol, |
| - 3 | mol% | *N*-stearoyl-D-erythro-sphingosylphosphorylcholine, |
| - 1 | mol% | 1,2-dioleoyl-sn-glycero-3-phosphoethanolamine-*N*-(lissamine rhodamine B Sulfonyl), and |
| - 1 | mol% | of a lipid-bound Spike protein ectodomain, wherein the Spike protein ectodomain binds to a cell surface receptor, |

wherein the lipoparticle has a diameter between 20 nm and 300 nm as measured by dynamic light scattering;
(b) contacting the lipoparticle with a sample, to bind the lipoparticle to the cells contained in the sample;
(c') detecting or determining fluorescence in the sample, thereby detecting or determining the total number of lipoparticles in the sample;
(c) washing the sample, thereby removing the lipoparticles not bound to the cells:
(d) detecting or determining fluorescence in the washed sample, thereby detecting or determining the number of lipoparticles bound to the cells in the sample.
In another embodiment, the inventive method for quantification of Spike mediated binding of the lipoparticle to a cell in a sample comprises the steps:
(a) providing a lipoparticle comprising: a lipid bilayer comprising

| | | |
|---|---|---|
| - 80-85 | mol% | 1,2-dioleoyl-sn-glycero-3-phosphocholine, |
| - 5-10 | mol% | 1,2-dioleoyl-sn-glycero-3-phosphoethanolamine, |
| - 5 | mol% | 1,2-dioleoyl-sn-glycero-3-phospho-(1'-myo-inositol), |
| - 3 | mol% | *N*-stearoyl-D-erythro-sphingosylphosphorylcholine, |
| - 1 | mol% | 1,2-dioleoyl-sn-glycero-3-phosphoethanolamine-*N*-(lissamine rhodamine B Sulfonyl), and |
| - 1 | mol% | of a lipid-bound Spike protein ectodomain, wherein the Spike protein ectodomain binds to a cell surface receptor, |

wherein the lipoparticle has a diameter between 20 nm and 300 nm as measured by dynamic light scattering;
(b) contacting the lipoparticle with a sample, to bind the lipoparticle to the cells contained in the sample;
(c') detecting or determining fluorescence in the sample, thereby detecting or determining the total number of lipoparticles in the sample;

(c) washing the sample, thereby removing the lipoparticles not bound to the cells:

(d) detecting or determining fluorescence in the washed sample, thereby detecting or determining the number of lipoparticles bound to the cells in the sample.

In another embodiment, the inventive method for quantification of Spike mediated binding of the lipoparticle to a cell in a sample comprises the steps:

(a) providing a lipoparticle comprising: a lipid bilayer comprising

| - 40 | mol% | 1,2-dioleoyl-sn-glycero-3-phosphocholine, |
| - 30 | mol% | 1,2-dioleoyl-sn-glycero-3-phosphoethanolamine, |
| - 22 | mol% | *N*-stearoyl-D-erythro-sphingosylphosphorylcholine, |
| - 5 | mol% | N-stearoyl-D-erythro-sphingosine, |
| - 1 | mol% | 1,2-dioleoyl-sn-glycero-3-phosphoethanolamine-*N*-(lissamine rhodamine B Sulfonyl), and |
| - 1 | mol% | of a lipid-bound Spike protein ectodomain, wherein the Spike protein ectodomain binds to a cell surface receptor, |

wherein the lipoparticle has a diameter between 20 nm and 300 nm as measured by dynamic light scattering;

(b) contacting the lipoparticle with a sample, to bind the lipoparticle to the cells contained in the sample;

(c') detecting or determining fluorescence in the sample, thereby detecting or determining the total number of lipoparticles in the sample;

(c) washing the sample, thereby removing the lipoparticles not bound to the cells:

(d) detecting or determining fluorescence in the washed sample, thereby detecting or determining the number of lipoparticles bound to the cells in the sample.

In the methods described herein, the lipoparticles have preferably a diameter between 80 nm and 140 nm as measured by dynamic light scattering. Preferably, the lipid-bound fluorophore is present in the lipid bilayer of the inventive lipoparticles in the amount of 1 mol%.

Preferably, the Spike protein ectodomain is bound via a His-Tag to Ni-NTA functionalized lipid, preferably the nickel salt of 1,2-dioleoyl-sn-glycero-3-[(N-(5-amino-1-carboxypentyl)iminodiacetic acid)succinyl].

In another embodiment, the inventive method for quantification of Spike mediated binding of the lipoparticle to a cell in a sample comprises the steps:

(a) providing a lipoparticle comprising: a lipid bilayer comprising

- between 40 and 85 mol percent of a phosphatidylcholine as neutral amphiphile,
- an anionic lipid,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering;

(b) contacting the lipoparticle with a sample, to bind the lipoparticle to the cells contained in the sample;

(c') detecting or determining fluorescence in the sample, thereby detecting or determining the total number of lipoparticles in the sample;

(c) washing the sample, thereby removing the lipoparticles not bound to the cells:

(d) detecting or determining fluorescence in the washed sample, thereby detecting or determining the number of lipoparticles bound to the cells in the sample,

wherein the Spike protein ectodomain is selected from the SARS-CoV-2 Spike protein ectodomain or a mutant thereof, SARS-CoV Spike protein ectodomain or a mutant thereof, MERS-CoV Spike protein ectodomain or a mutant thereof, human immunodeficiency virus type 1 envelope Spike protein ectodomain or a mutant thereof, large Hepatitis B virus envelope Spike protein ectodomain or a mutant thereof, Hepatitis C virus or a mutant thereof, Herpes simplex virus 1 glycoprotein B, C, D, H, or L ectodomain or a mutant thereof, Herpes simplex virus 2 glycoprotein B, C, D, H, or L ectodomain or a mutant thereof, Ebolavirus glycoprotein ectodomain or a mutant thereof, Marburg virus glycoprotein ectodomain or a mutant thereof, Influenza virus A hemagglutinin ectodomain or mutant thereof, Influenza virus B hemagglutinin ectodomain or mutant thereof, Influenza virus C hemagglutinin ectodomain or mutant thereof, Influenza virus D hemagglutinin ectodomain or mutant thereof, Dengue virus E envelope protein ectodomain or a mutant thereof, Zika virus Spike protein ectodomain or a mutant thereof, a mutant thereof; preferably selected from SARS-CoV-2 Spike protein ectodomain or a mutant thereof, SARS-CoV Spike protein ectodomain or a mutant thereof, MERS-CoV Spike protein ectodomain or a mutant thereof, HIV-1 gp 120 protein, Hepatitis B Surface Antigen adw subtype 2 (HBVsAG), Hepatitis C E1 protein, Ebolavirus glycoprotein, Marburg virus glycoprotein (AMARV GP), Influenza virus A hemagglutinin H1 protein, and Dengue virus 2 E-glycoprotein.

In another embodiment, the inventive method for quantification of Spike mediated binding of the lipoparticle to a cell in a sample comprises the steps:

(a) providing a lipoparticle comprising: a lipid bilayer comprising

- between 40 and 85 mol percent of a phosphatidylcholine as neutral amphiphile,
- an anionic lipid,
- a lipid-bound fluorophore, and
- at least 1 mol percent of a lipid-bound SARS-CoV-2 Spike protein ectodomain mutant homotrimer, wherein the homotrimer binds to a cell surface receptor,

wherein the lipoparticle has a diameter between 20 nm and 300 nm as measured by dynamic light scattering;
(b) contacting the lipoparticle with a sample, to bind the lipoparticle to the cells contained in the sample;
(c') detecting or determining fluorescence in the sample, thereby detecting or determining the total number of lipoparticles in the sample;
(c) washing the sample, thereby removing the lipoparticles not bound to the cells:
(d) detecting or determining fluorescence in the washed sample, thereby detecting or determining the number of lipoparticles bound to the cells in the sample.

Preferably, each monomer of the SARS-CoV-2 Spike protein ectodomain comprises an amino acid sequence identical to the sequence as set forth in SEQ ID NO 5 and wherein each monomer comprises further a single point mutation at one of the following sites: L4, T6, P12, D66, D124, R176, D201, R232, K403, L411, E470, N487, A556, D600, H641, Q663, P667, A684, T699, S965, T1010, and D1101. More preferably, each monomer of the SARS-CoV-2 Spike protein ectodomain mutant homotrimer comprises an amino acid sequence identical to one of the sequences as set forth in SEQ ID NOs 6 to 29.

In another embodiment, the inventive method for quantification of Spike mediated binding of the lipoparticle to a cell in a sample comprises the steps:

(a) providing a lipoparticle comprising: a lipid bilayer comprising

- between 40 and 85 mol percent of a phosphatidylcholine as neutral amphiphile,
- an anionic lipid,
- a lipid-bound fluorophore, and
- at least 1 mol percent of a lipid-bound Spike protein ectodomain, wherein the Spike protein ectodomain binds to a cell surface receptor and is devoid of free fatty acids,

wherein the lipoparticle has a diameter between 20 nm and 300 nm as measured by dynamic light scattering;
(b) contacting the lipoparticle with a sample, to bind the lipoparticle to the cells contained in the sample;
(c') detecting or determining fluorescence in the sample, thereby detecting or determining the total number of lipoparticles in the sample;
(c) washing the sample, thereby removing the lipoparticles not bound to the cells:
(d) detecting or determining fluorescence in the washed sample, thereby detecting or determining the number of lipoparticles bound to the cells in the sample.

In another embodiment, the inventive method for quantification of Spike mediated binding of the lipoparticle to a cell in a sample comprises the steps:

(a) providing a lipoparticle comprising: a lipid bilayer comprising

- between 40 and 85 mol percent of a phosphatidylcholine as neutral amphiphile,
- an anionic lipid,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering;

(b) contacting the lipoparticle with a sample, to bind the lipoparticle to the cells contained in the sample;

(c') detecting or determining fluorescence in the sample, thereby detecting or determining the total number of lipoparticles in the sample;

(c) washing the sample, thereby removing the lipoparticles not bound to the cells:

(d) detecting or determining fluorescence in the washed sample, thereby detecting or determining the number of lipoparticles bound to the cells in the sample;

wherein the cell surface receptor is selected from angiotensin-converting enzyme 2, DPP4, CD4, CCR5, NTCP, CD81, SR-BI, DC-SIGN, L-SIGN, heparan sulphate proteoglycans, asialoglycoprotein receptors, herpesvirus entry mediator, nectin-1, nectin-2, Sialic acid-containing receptors, nLc$_4$Cer, HSP70/HSP90, GRP78, Laminin receptor and TIM-1. Preferably, the cell surface receptor is angiotensin-converting enzyme 2 (ACE2).

A further aspect of the present invention is directed to a **method for quantification of binding of a lipoparticle to an antibody** that specifically binds to the Spike protein in a sample comprising the steps:

(a) providing a lipoparticle comprising: a lipid bilayer comprising

- between 40 and 85 mol percent of a phosphatidylcholine as neutral amphiphile,
- at least one anionic lipid,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering;

(b) contacting the lipoparticle with a sample, to bind the synthetic lipoparticle to the antibody contained in the sample;

(c) detecting or determining fluorescence in the sample, thereby detecting or determining an amount of antibody that binds to the lipoparticle in the sample.

In another embodiment, the inventive method for quantification of binding of a lipoparticle to an antibody in a sample comprises the steps:

(a) providing a lipoparticle comprising: a lipid bilayer comprising

- between 40 and 85 mol percent of a phosphatidylcholine as neutral amphiphile,
- a phosphatidylserine,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering;

(b) contacting the lipoparticle with a sample, to bind the synthetic lipoparticle to the antibody contained in the sample;

(c) detecting or determining fluorescence in the sample, thereby detecting or determining an amount of antibody that binds to the lipoparticle in the sample.

In another embodiment, the inventive method for quantification of binding of a lipoparticle to an antibody in a sample comprises the steps:

(a) providing a lipoparticle comprising: a lipid bilayer comprising

- between 40 and 85 mol percent of a phosphatidylcholine as neutral amphiphile,
- a phosphatidylglycerol,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering;

(b) contacting the lipoparticle with a sample, to bind the synthetic lipoparticle to the antibody contained in the sample;

(c) detecting or determining fluorescence in the sample, thereby detecting or determining an amount of antibody that binds to the lipoparticle in the sample.

In another embodiment, the inventive method for quantification of binding of a lipoparticle to an antibody in a sample comprises the steps:

(a) providing a lipoparticle comprising: a lipid bilayer comprising

- between 40 and 85 mol percent of a phosphatidylcholine as neutral amphiphile,
- a phosphatidylserine,
- a phosphatidylglycerol,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering;
(b) contacting the lipoparticle with a sample, to bind the synthetic lipoparticle to the antibody contained in the sample;
(c) detecting or determining fluorescence in the sample, thereby detecting or determining an amount of antibody that binds to the lipoparticle in the sample.

In another embodiment, the inventive method for quantification of binding of a lipoparticle to an antibody in a sample comprises the steps:

(a) providing a lipoparticle comprising: a lipid bilayer comprising

- between 40 and 85 mol percent of a phosphatidylcholine as neutral amphiphile,
- a phosphatidylserine,
- a phosphatidylinositol,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering;
(b) contacting the lipoparticle with a sample, to bind the synthetic lipoparticle to the antibody contained in the sample;
(c) detecting or determining fluorescence in the sample, thereby detecting or determining an amount of antibody that binds to the lipoparticle in the sample.

In another embodiment, the inventive method for quantification of binding of a lipoparticle to an antibody in a sample comprises the steps:

(a) providing a lipoparticle comprising: a lipid bilayer comprising

- between 40 and 85 mol percent of DOPC,
- DOPS,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering;
(b) contacting the lipoparticle with a sample, to bind the synthetic lipoparticle to the antibody contained in the sample;
(c) detecting or determining fluorescence in the sample, thereby detecting or determining an amount of antibody that binds to the lipoparticle in the sample.

In another embodiment, the inventive method for quantification of binding of a lipoparticle to an antibody in a sample comprises the steps:

(a) providing a lipoparticle comprising: a lipid bilayer comprising

- between 40 and 85 mol percent of a phosphatidylcholine,

- a phosphatidylethanolamine,
- a phosphatidylserine or a phosphatidylglycerol or a phosphatidylinositol,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering;
(b) contacting the lipoparticle with a sample, to bind the synthetic lipoparticle to the antibody contained in the sample;
(c) detecting or determining fluorescence in the sample, thereby detecting or determining an amount of antibody that binds to the lipoparticle in the sample.

In another embodiment, the inventive method for quantification of binding of a lipoparticle to an antibody in a sample comprises the steps:

(a) providing a lipoparticle comprising: a lipid bilayer comprising

- a phosphatidylethanolamine
- a phosphatidylinositol
- a cholesterol
- a sphingosylphosphorylcholine,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering;
(b) contacting the lipoparticle with a sample, to bind the synthetic lipoparticle to the antibody contained in the sample;
(c) detecting or determining fluorescence in the sample, thereby detecting or determining an amount of antibody that binds to the lipoparticle in the sample.

In another embodiment, the inventive method for quantification of binding of a lipoparticle to an antibody in a sample comprises the steps:
(a) providing a lipoparticle comprising: a lipid bilayer comprising

| - 40-50 | mol% | 1,2-dioleoyl-sn-glycero-3-phosphocholine, |
| - 15-25 | mol% | 1,2-dioleoyl-sn-glycero-3-phosphoethanolamine, |
| - 2-4 | mol% | 1,2-dioleoyl-sn-glycero-3-phospho-l-serine, |
| - 12 | mol% | 1,2-dioleoyl-sn-glycero-3-phospho-(1'-myo-inositol), |
| - 14 | mol% | cholesterol, |
| - 3 | mol% | N-stearoyl-D-erythro-sphingosylphosphorylcholine, |
| - 1 | mol% | 1,2-dioleoyl-sn-glycero-3-phosphoethanolamine-N-(lissamine rhodamine B Sulfonyl), and |
| - 1 | mol% | of a lipid-bound Spike protein ectodomain, wherein the Spike protein ectodomain binds to a cell surface receptor, |

wherein the lipoparticle has a diameter between 20 nm and 300 nm as measured by dynamic light scattering;
(b) contacting the lipoparticle with a sample, to bind the synthetic lipoparticle to the antibody contained in the sample;
(c) detecting or determining fluorescence in the sample, thereby detecting or determining an amount of antibody that binds to the lipoparticle in the sample.
In another embodiment, the inventive method for quantification of binding of a lipoparticle to an antibody in a sample comprises the steps:
(a) providing a lipoparticle comprising: a lipid bilayer comprising

| - 80-85 | mol% | 1,2-dioleoyl-sn-glycero-3-phosphocholine, |
| - 5-10 | mol% | 1,2-dioleoyl-sn-glycero-3-phosphoethanolamine, |
| - 5 | mol% | 1,2-dioleoyl-sn-glycero-3-phospho-(1'-myo-inositol), |
| - 3 | mol% | N-stearoyl-D-erythro-sphingosylphosphorylcholine, |
| - 1 | mol% | 1,2-dioleoyl-sn-glycero-3-phosphoethanolamine-N-(lissamine rhodamine B Sulfonyl), and |

(continued)

| | | |
|---|---|---|
| - 1 | mol% | of a lipid-bound Spike protein ectodomain, wherein the Spike protein ectodomain binds to a cell surface receptor, |

wherein the lipoparticle has a diameter between 20 nm and 300 nm as measured by dynamic light scattering;
(b) contacting the lipoparticle with a sample, to bind the synthetic lipoparticle to the antibody contained in the sample;
(c) detecting or determining fluorescence in the sample, thereby detecting or determining an amount of antibody that binds to the lipoparticle in the sample.

In another embodiment, the inventive method for quantification of binding of a lipoparticle to an antibody in a sample comprises the steps:

(a) providing a lipoparticle comprising: a lipid bilayer comprising

| | | |
|---|---|---|
| - 40 | mol% | 1,2-dioleoyl-sn-glycero-3-phosphocholine, |
| - 30 | mol% | 1,2-dioleoyl-sn-glycero-3-phosphoethanolamine, |
| - 22 | mol% | *N*-stearoyl-D-erythro-sphingosylphosphorylcholine, |
| - 5 | mol% | N-stearoyl-D-erythro-sphingosine, |
| - 1 | mol% | 1,2-dioleoyl-sn-glycero-3-phosphoethanolamine-*N*-(lissamine rhodamine B Sulfonyl), and |
| - 1 | mol% | of a lipid-bound Spike protein ectodomain, wherein the Spike protein ectodomain binds to a cell surface receptor, |

wherein the lipoparticle has a diameter between 20 nm and 300 nm as measured by dynamic light scattering;
(b) contacting the lipoparticle with a sample, to bind the synthetic lipoparticle to the antibody contained in the sample;
(c) detecting or determining fluorescence in the sample, thereby detecting or determining an amount of antibody that binds to the lipoparticle in the sample.

In the methods described herein, the lipoparticles have preferably a diameter between 80 nm and 140 nm as measured by dynamic light scattering. Preferably, the lipid-bound fluorophore is present in the lipid bilayer of the inventive lipoparticles in the amount of 1 mol%.

Preferably, the Spike protein ectodomain is bound via a His-Tag to Ni-NTA functionalized lipid, preferably the nickel salt of 1,2-dioleoyl-sn-glycero-3-[(N-(5-amino-1-carboxypentyl)iminodiacetic acid)succinyl].

In another embodiment, the inventive method for quantification of binding of a lipoparticle to an antibody in a sample comprises the steps:

(a) providing a lipoparticle comprising: a lipid bilayer comprising

- between 40 and 85 mol percent of a phosphatidylcholine as neutral amphiphile,
- an anionic lipid,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering;
(b) contacting the lipoparticle with a sample, to bind the synthetic lipoparticle to the antibody contained in the sample;
(c) detecting or determining fluorescence in the sample, thereby detecting or determining an amount of antibody that binds to the lipoparticle in the sample;

wherein the Spike protein ectodomain is selected from the SARS-CoV-2 Spike protein ectodomain or a mutant thereof, SARS-CoV Spike protein ectodomain or a mutant thereof, MERS-CoV Spike protein ectodomain or a mutant thereof, human immunodeficiency virus type 1 envelope Spike protein ectodomain or a mutant thereof, large Hepatitis B virus envelope Spike protein ectodomain or a mutant thereof, Hepatitis C virus or a mutant thereof, Herpes simplex virus 1 glycoprotein B, C, D, H, or L ectodomain or a mutant thereof, Herpes simplex virus 2 glycoprotein B, C, D, H, or L ectodomain or a mutant thereof, Ebolavirus glycoprotein ectodomain or a mutant thereof, Marburg virus glycoprotein ectodomain or a mutant thereof, Influenza virus A hemagglutinin ectodomain or mutant thereof, Influenza virus B hemagglutinin ectodomain or mutant thereof, Influenza virus C hemagglutinin ectodomain or mutant thereof, Influenza virus D hemagglutinin ectodomain or mutant thereof, Dengue virus E envelope protein ectodomain or a mutant thereof, Zika virus Spike protein ectodomain or a mutant thereof, a mutant thereof; preferably selected from SARS-CoV-2 Spike protein ectodomain or a mutant thereof, SARS-CoV Spike protein ectodomain or a mutant thereof, MERS-CoV Spike protein ectodomain or a mutant thereof, HIV-1 gp 120 protein, Hepatitis B Surface Antigen adw subtype 2 (HBVsAG), Hepatitis C E1 protein, Ebolavirus glycoprotein, Marburg virus glycoprotein (AMARV GP), Influenza virus A hemagglutinin H1 protein, and Dengue virus 2 E-glycoprotein.

In another embodiment, the inventive method for quantification of binding of a lipoparticle to an antibody in a sample comprises the steps:

(a) providing a lipoparticle comprising: a lipid bilayer comprising

- between 40 and 85 mol percent of a phosphatidylcholine as neutral amphiphile,
- an anionic lipid,
- a lipid-bound fluorophore, and
- at least 1 mol percent of a lipid-bound SARS-CoV-2 Spike protein ectodomain mutant homotrimer, wherein the homotrimer binds to a cell surface receptor,

wherein the lipoparticle has a diameter between 20 nm and 300 nm as measured by dynamic light scattering;
(b) contacting the lipoparticle with a sample, to bind the synthetic lipoparticle to the antibody contained in the sample;
(c) detecting or determining fluorescence in the sample, thereby detecting or determining an amount of antibody that binds to the lipoparticle in the sample.

Preferably, each monomer of the SARS-CoV-2 Spike protein ectodomain comprises an amino acid sequence identical to the sequence as set forth in SEQ ID NO 5 and wherein each monomer comprises further a single point mutation at one of the following sites: L4, T6, P12, D66, D124, R176, D201, R232, K403, L411, E470, N487, A556, D600, H641, Q663, P667, A684, T699, S965, T1010, and D1101. More preferably, each monomer of the SARS-CoV-2 Spike protein ectodomain mutant homotrimer comprises an amino acid sequence identical to one of the sequences as set forth in SEQ ID NOs 6 to 29.

In another embodiment, the inventive method for quantification of binding of a lipoparticle to an antibody in a sample comprises the steps:

(a) providing a lipoparticle comprising: a lipid bilayer comprising

- between 40 and 85 mol percent of a phosphatidylcholine as neutral amphiphile,
- an anionic lipid,
- a lipid-bound fluorophore, and
- at least 1 mol percent of a lipid-bound Spike protein ectodomain, wherein the Spike protein ectodomain binds to a cell surface receptor and is devoid of free fatty acids,

wherein the lipoparticle has a diameter between 20 nm and 300 nm as measured by dynamic light scattering;
(b) contacting the lipoparticle with a sample, to bind the synthetic lipoparticle to the antibody contained in the sample;
(c) detecting or determining fluorescence in the sample, thereby detecting or determining an amount of antibody that binds to the lipoparticle in the sample.

In another embodiment, the inventive method for quantification of binding of a lipoparticle to an antibody in a sample comprises the steps:

(a) providing a lipoparticle comprising: a lipid bilayer comprising

- between 40 and 85 mol percent of a phosphatidylcholine as neutral amphiphile,
- an anionic lipid,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering;
(b) contacting the lipoparticle with a sample, to bind the synthetic lipoparticle to the antibody contained in the sample;
(c) detecting or determining fluorescence in the sample, thereby detecting or determining an amount of antibody that binds to the lipoparticle in the sample;

wherein the cell surface receptor is selected from angiotensin-converting enzyme 2, DPP4, CD4, CCR5, NTCP, CD81, SR-BI, DC-SIGN, L-SIGN, heparan sulphate proteoglycans, asialoglycoprotein receptors, herpesvirus entry mediator, nectin-1, nectin-2, Sialic acid-containing receptors, $nLc_4Cer$, HSP70/HSP90, GRP78, Laminin receptor and TIM-1. Preferably, the cell surface receptor is angiotensin-converting enzyme 2 (ACE2).

A further aspect of the present invention is directed to a **method for producing a lipoparticle** according to the present invention. The method comprises the steps:

(a) providing a rehydrated lipid solution,
(b) extruding said rehydrated lipid solution through a porous membrane at least two times, thereby producing lipoparticles.

A further aspect of the present invention is directed to a **method for screening for and/or identifying an agent or molecule that can block or inhibit Spike mediated binding of a synthetic lipoparticle** to a cell in a sample comprising the steps:

(a1) providing a lipoparticle comprising: a lipid bilayer comprising

- between 40 and 85 mol percent of a phosphatidylcholine as neutral amphiphile,
- at least one anionic lipid,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering;
(a2) providing a test agent or a test molecule;

(b) mixing or combining the test agent or test molecule with a cells containing sample
(c) contacting the lipoparticle with the sample comprising the test agent or test molecule, to bind the lipoparticle to the cells contained in the sample;
(d) washing the sample, thereby removing the lipoparticles not bound to the cells;
(e) detecting or determining if the lipoparticles are bound to the cells in the sample by detecting or determining fluorescence in the washed sample.

In one embodiment, the inventive method for screening for and/or identifying an agent or molecule that can block or inhibit Spike mediated binding of a synthetic lipoparticle to a cell in a sample comprises the steps:

(a1) providing a lipoparticle comprising: a lipid bilayer comprising

- between 40 and 85 mol percent of a phosphatidylcholine as neutral amphiphile,
- a phosphatidylglycerol,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering;
(a2) providing a test agent or a test molecule;

(b) mixing or combining the test agent or test molecule with a cells containing sample
(c) contacting the lipoparticle with the sample comprising the test agent or test molecule, to bind the lipoparticle to the cells contained in the sample;
(d) washing the sample, thereby removing the lipoparticles not bound to the cells;
(e) detecting or determining if the lipoparticles are bound to the cells in the sample by detecting or determining fluorescence in the washed sample.

In one embodiment, the inventive method for screening for and/or identifying an agent or molecule that can block or inhibit Spike mediated binding of a synthetic lipoparticle to a cell in a sample comprises the steps:

(a1) providing a lipoparticle comprising: a lipid bilayer comprising

- between 40 and 85 mol percent of a phosphatidylcholine as neutral amphiphile,
- a phosphatidylserine,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering;
(a2) providing a test agent or a test molecule;

(b) mixing or combining the test agent or test molecule with a cells containing sample
(c) contacting the lipoparticle with the sample comprising the test agent or test molecule, to bind the lipoparticle to the cells contained in the sample;
(d) washing the sample, thereby removing the lipoparticles not bound to the cells;
(e) detecting or determining if the lipoparticles are bound to the cells in the sample by detecting or determining fluorescence in the washed sample.

In one embodiment, the inventive method for screening for and/or identifying an agent or molecule that can block or inhibit Spike mediated binding of a synthetic lipoparticle to a cell in a sample comprises the steps:

(a1) providing a lipoparticle comprising: a lipid bilayer comprising

- between 40 and 85 mol percent of a phosphatidylcholine as neutral amphiphile,
- a phosphatidylserine,
- a phosphatidylglycerol,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering;
(a2) providing a test agent or a test molecule;
(b) mixing or combining the test agent or test molecule with a cells containing sample
(c) contacting the lipoparticle with the sample comprising the test agent or test molecule, to bind the lipoparticle to the cells contained in the sample;
(d) washing the sample, thereby removing the lipoparticles not bound to the cells;
(e) detecting or determining if the lipoparticles are bound to the cells in the sample by detecting or determining fluorescence in the washed sample.

In one embodiment, the inventive method for screening for and/or identifying an agent or molecule that can block or inhibit Spike mediated binding of a synthetic lipoparticle to a cell in a sample comprises the steps:

(a1) providing a lipoparticle comprising: a lipid bilayer comprising
-- between 40 and 85 mol percent of a phosphatidylcholine as neutral amphiphile,

- a phosphatidylserine,
- a phosphatidylinositol,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering;
(a2) providing a test agent or a test molecule;

(b) mixing or combining the test agent or test molecule with a cells containing sample
(c) contacting the lipoparticle with the sample comprising the test agent or test molecule, to bind the lipoparticle to the cells contained in the sample;
(d) washing the sample, thereby removing the lipoparticles not bound to the cells;
(e) detecting or determining if the lipoparticles are bound to the cells in the sample by detecting or determining fluorescence in the washed sample.

In one embodiment, the inventive method for screening for and/or identifying an agent or molecule that can block or inhibit Spike mediated binding of a synthetic lipoparticle to a cell in a sample comprises the steps:

(a1) providing a lipoparticle comprising: a lipid bilayer comprising

- between 40 and 85 mol percent of DOPC,
- DOPS,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering;
(a2) providing a test agent or a test molecule;
(b) mixing or combining the test agent or test molecule with a cells containing sample
(c) contacting the lipoparticle with the sample comprising the test agent or test molecule, to bind the lipoparticle to the cells contained in the sample;
(d) washing the sample, thereby removing the lipoparticles not bound to the cells;
(e) detecting or determining if the lipoparticles are bound to the cells in the sample by detecting or determining fluorescence in the washed sample.

In one embodiment, the inventive method for screening for and/or identifying an agent or molecule that can block or inhibit Spike mediated binding of a synthetic lipoparticle to a cell in a sample comprises the steps:

(a1) providing a lipoparticle comprising: a lipid bilayer comprising
-- between 40 and 85 mol percent of a phosphatidylcholine,

- a phosphatidylethanolamine,
- a phosphatidylserine or a phosphatidylglycerol or a phosphatidylinositol,
- a lipid-bound fluorophore, and

- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering;
(a2) providing a test agent or a test molecule;
(b) mixing or combining the test agent or test molecule with a cells containing sample
(c) contacting the lipoparticle with the sample comprising the test agent or test molecule, to bind the lipoparticle to the cells contained in the sample;
(d) washing the sample, thereby removing the lipoparticles not bound to the cells;
(e) detecting or determining if the lipoparticles are bound to the cells in the sample by detecting or determining fluorescence in the washed sample.

In one embodiment, the inventive method for screening for and/or identifying an agent or molecule that can block or inhibit Spike mediated binding of a synthetic lipoparticle to a cell in a sample comprises the steps:

(a1) providing a lipoparticle comprising: a lipid bilayer comprising

- a phosphatidylethanolamine
- a phosphatidylinositol
- a cholesterol
- a sphingosylphosphorylcholine,
- a lipid-bound fluorophore, and
- at least 1 mol percent of a lipid-bound Spike protein ectodomain, wherein the Spike protein ectodomain binds to a cell surface receptor,

wherein the lipoparticle has a diameter between 20 nm and 300 nm as measured by dynamic light scattering;
(a2) providing a test agent or a test molecule;
(b) mixing or combining the test agent or test molecule with a cells containing sample

(c) contacting the lipoparticle with the sample comprising the test agent or test molecule, to bind the lipoparticle to the cells contained in the sample;

(d) washing the sample, thereby removing the lipoparticles not bound to the cells;

(e) detecting or determining if the lipoparticles are bound to the cells in the sample by detecting or determining fluorescence in the washed sample.

In one embodiment, the inventive method for screening for and/or identifying an agent or molecule that can block or inhibit Spike mediated binding of a synthetic lipoparticle to a cell in a sample comprises the steps:

(a1) providing a lipoparticle comprising: a lipid bilayer comprising

| | | |
|---|---|---|
| - 40-50 | mol% | 1,2-dioleoyl-sn-glycero-3-phosphocholine, |
| - 15-25 | mol% | 1,2-dioleoyl-sn-glycero-3-phosphoethanolamine, |
| - 2-4 | mol% | 1,2-dioleoyl-sn-glycero-3-phospho-l-serine, |
| - 12 | mol% | 1,2-dioleoyl-sn-glycero-3-phospho-(1'-myo-inositol), |
| - 14 | mol% | cholesterol, |
| - 3 | mol% | *N*-stearoyl-d-erythro-sphingosylphosphorylcholine, |
| - 1 | mol% | 1,2-dioleoyl-sn-glycero-3-phosphoethanolamine-*N*-(lissamine rhodamine B Sulfonyl), and |
| - 1 | mol% | of a lipid-bound Spike protein ectodomain, wherein the Spike protein ectodomain binds to a cell surface receptor, |

wherein the lipoparticle has a diameter between 20 nm and 300 nm as measured by dynamic light scattering;

(a2) providing a test agent or a test molecule;

(b) mixing or combining the test agent or test molecule with a cells containing sample

(c) contacting the lipoparticle with the sample comprising the test agent or test molecule, to bind the lipoparticle to the cells contained in the sample;

(d) washing the sample, thereby removing the lipoparticles not bound to the cells;

(e) detecting or determining if the lipoparticles are bound to the cells in the sample by detecting or determining fluorescence in the washed sample.

In one embodiment, the inventive method for screening for and/or identifying an agent or molecule that can block or inhibit Spike mediated binding of a synthetic lipoparticle to a cell in a sample comprises the steps:

(a1) providing a lipoparticle comprising: a lipid bilayer comprising

| | | |
|---|---|---|
| - 80-85 | mol% | 1,2-dioleoyl-sn-glycero-3-phosphocholine, |
| - 5-10 | mol% | 1,2-dioleoyl-sn-glycero-3-phosphoethanolamine, |
| - 5 | mol% | 1,2-dioleoyl-sn-glycero-3-phospho-(1'-myo-inositol), |
| - 3 | mol% | *N*-stearoyl-d-erythro-sphingosylphosphorylcholine, |
| - 1 | mol% | 1,2-dioleoyl-sn-glycero-3-phosphoethanolamine-*N*-(lissamine rhodamine B Sulfonyl), and |
| - 1 | mol% | of a lipid-bound Spike protein ectodomain, wherein the Spike protein ectodomain binds to a cell surface receptor, |

wherein the lipoparticle has a diameter between 20 nm and 300 nm as measured by dynamic light scattering;

(a2) providing a test agent or a test molecule;

(b) mixing or combining the test agent or test molecule with a cells containing sample

(c) contacting the lipoparticle with the sample comprising the test agent or test molecule, to bind the lipoparticle to the cells contained in the sample;

(d) washing the sample, thereby removing the lipoparticles not bound to the cells;

(e) detecting or determining if the lipoparticles are bound to the cells in the sample by detecting or determining fluorescence in the washed sample.

In one embodiment, the inventive method for screening for and/or identifying an agent or molecule that can block or inhibit Spike mediated binding of a synthetic lipoparticle to a cell in a sample comprises the steps:

(a1) providing a lipoparticle comprising: a lipid bilayer comprising

| | | |
|---|---|---|
| - 40 | mol% | 1,2-dioleoyl-sn-glycero-3-phosphocholine, |
| - 30 | mol% | 1,2-dioleoyl-sn-glycero-3-phosphoethanolamine, |
| - 22 | mol% | *N*-stearoyl-d-erythro-sphingosylphosphorylcholine, |

(continued)

| | | |
|---|---|---|
| - 5 | mol% | N-stearoyl-d-erythro-sphingosine, |
| - 1 | mol% | 1,2-dioleoyl-sn-glycero-3-phosphoethanolamine-*N*-(lissamine rhodamine B Sulfonyl), and |
| - 1 | mol% | of a lipid-bound Spike protein ectodomain, wherein the Spike protein ectodomain binds to a cell surface receptor, |

wherein the lipoparticle has a diameter between 20 nm and 300 nm as measured by dynamic light scattering;

(a2) providing a test agent or a test molecule;

(b) mixing or combining the test agent or test molecule with a cells containing sample

(c) contacting the lipoparticle with the sample comprising the test agent or test molecule, to bind the lipoparticle to the cells contained in the sample;

(d) washing the sample, thereby removing the lipoparticles not bound to the cells;

(e) detecting or determining if the lipoparticles are bound to the cells in the sample by detecting or determining fluorescence in the washed sample.

In the methods described herein, the lipoparticles have preferably a diameter between 80 nm and 140 nm as measured by dynamic light scattering. Preferably, the lipid-bound fluorophore is present in the lipid bilayer of the inventive lipoparticles in the amount of 1 mol%.

Preferably, the Spike protein ectodomain is bound via a His-Tag to Ni-NTA functionalized lipid, preferably the nickel salt of 1,2-dioleoyl-sn-glycero-3-[(N-(5-amino-1-carboxypentyl)iminodiacetic acid)succinyl].

Preferably, the Spike protein ectodomain is selected from the SARS-CoV-2 Spike protein ectodomain or a mutant thereof, SARS-CoV Spike protein ectodomain or a mutant thereof, MERS-CoV Spike protein ectodomain or a mutant thereof, human immunodeficiency virus type 1 envelope Spike protein ectodomain or a mutant thereof, large Hepatitis B virus envelope Spike protein ectodomain or a mutant thereof, Hepatitis C virus or a mutant thereof, Herpes simplex virus 1 glycoprotein B, C, D, H, or L ectodomain or a mutant thereof, Herpes simplex virus 2 glycoprotein B, C, D, H, or L ectodomain or a mutant thereof, Ebolavirus glycoprotein ectodomain or a mutant thereof, Marburg virus glycoprotein ectodomain or a mutant thereof, Influenza virus A hemagglutinin ectodomain or mutant thereof, Influenza virus B hemagglutinin ectodomain or mutant thereof, Influenza virus C hemagglutinin ectodomain or mutant thereof, Influenza virus D hemagglutinin ectodomain or mutant thereof, Dengue virus E envelope protein ectodomain or a mutant thereof, Zika virus Spike protein ectodomain or a mutant thereof, a mutant thereof.

Even more preferably, the Spike protein ectodomain is selected from SARS-CoV-2 Spike protein ectodomain or a mutant thereof, SARS-CoV Spike protein ectodomain or a mutant thereof, MERS-CoV Spike protein ectodomain or a mutant thereof, HIV-1 gp 120 protein, Hepatitis B Surface Antigen adw subtype 2 (HBVsAG), Hepatitis C E1 protein, Ebolavirus glycoprotein, Marburg virus glycoprotein (AMARV GP), Influenza virus A hemagglutinin H1 protein, and Dengue virus 2 E-glycoprotein.

## Description of Figures

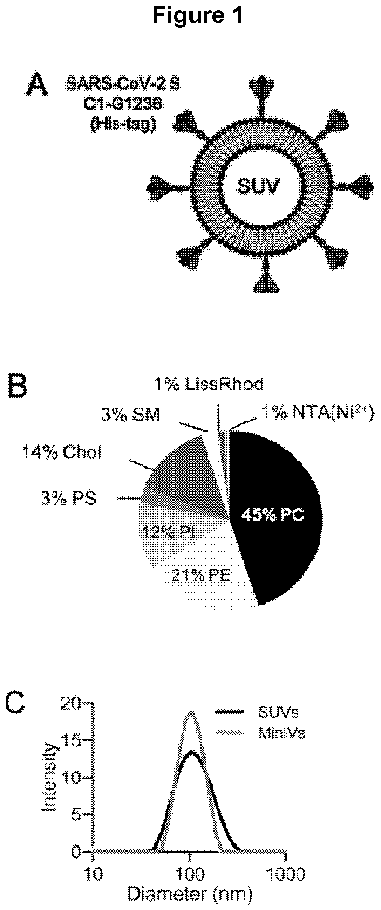

Fig. 1: (**A**) Schematic illustration of lipoparticles based on SUVs with SARS-CoV-2 S ectodomains, immobilized via their His-tag. (**B**) Lipid formulation of SUVs derived from the endoplasmic-reticulum-Golgi intermediate compartment (ERGIC) with NTA-functionalized and fluorescent lipids. (**C**) lipoparticle and SUV size distribution analysis by dynamic light scattering.

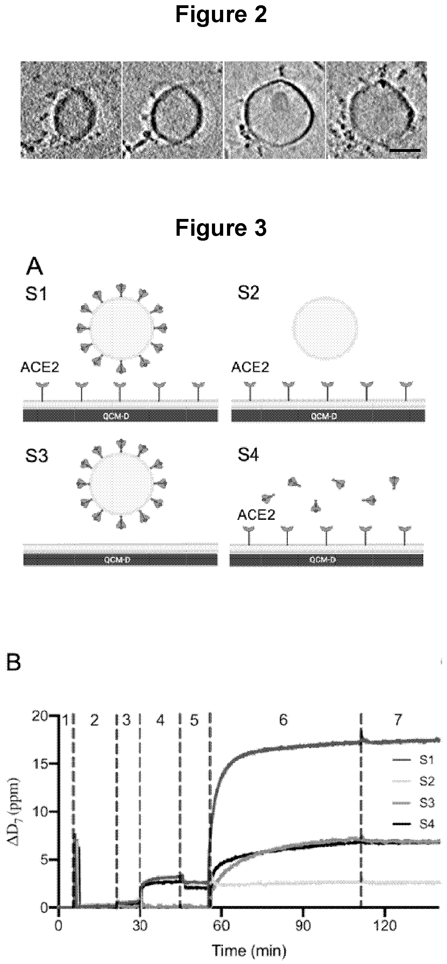

Fig. 2: shows exemplarily cryo-EM tomography slices of lipoparticles with immobilized S on the membrane. Scale bar is 50 nm.

Fig. 3: QCM-D Assessment of lipoparticle ACE2 binding. (**A**) Schematic illustration of experimental conditions (S1-S4) tested for binding of lipoparticles with and without S on supported lipid bilayer-(SLB) covered QCM-D quartz crystals presenting recombinant human ACE2 ectodomains. (**B**) Changes in dissipation in 7th harmony ((Df norm= Df n/n, with n being the seventh overtone (n=7)) over the whole experimental analysis for S1-S4. Phases correspond to: 1. Crystal equilibration in PBS; 2. Addition of SUVs and SLB formation with vesicles containing NTA($Ni^{2+}$) lipids; 3. PBS wash; 4. Addition of histidine-tagged recombinant human ACE2 ectodomains or PBS; 5. PBS wash; 6. Addition of lipoparticles, SUVs or soluble Spike protein, 7. PBS wash.

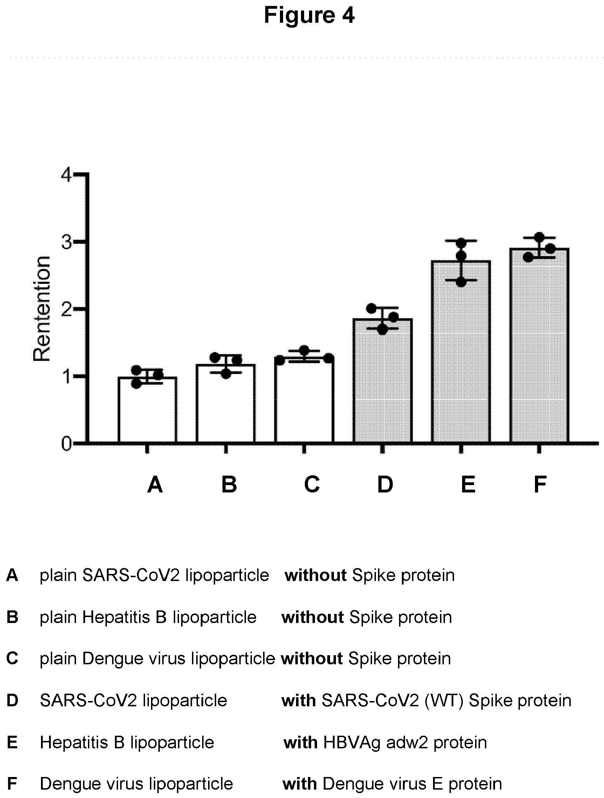

Fig. 4: Retention assay of inventive lipoparticles having a lipid constitution similar to **A:** SARS-CoV2 without surface protein, **B:** Hepatitis B virus without surface protein, **C:** Dengue virus without surface protein, **D:** SARS-CoV2 with Spike protein, **E:** Hepatitis B virus with HBV AG adw2 protein, and **F:** Dengue virus with Dengue virus E protein.

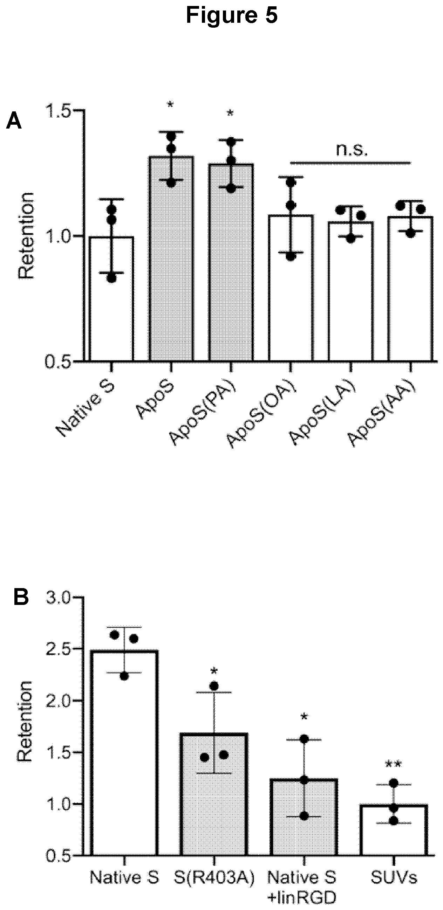

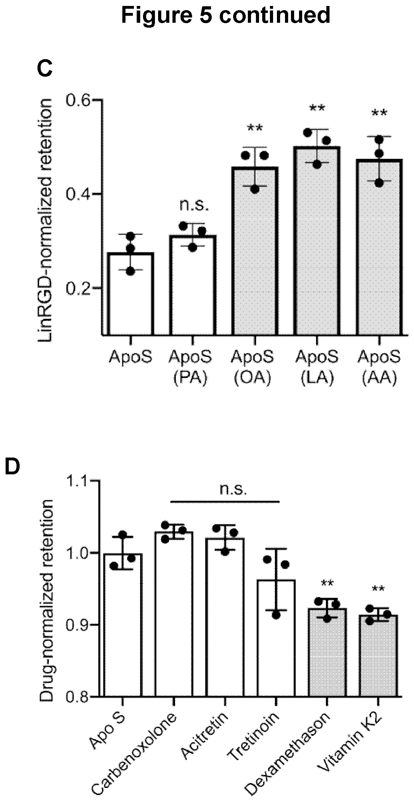

**Fig. 5:** (**A**) Native S-normalized retention assay for lipoparticles presenting apo SARS-CoV-2 Spike protein, loaded with 1 μM free fatty acids, after 24 h incubation with MCF7 cells. (**B**) SUV-normalized retention assay for lipoparticles presenting native SARS-CoV-2, R403A mutated SARS-CoV-2 without RGD motif or native SARS-CoV-2 incubated with 20 μM linRGD for integrin blocking. Retention was measured after 24 h incubation with MCF7 cells. (**C**) LinRGD-normalized retention assays for lipoparticles presenting free fatty acids-loaded apo SARS-CoV-2 after 24 h incubation with MCF7 cells. (**D**) Drug-normalized retention assay for apo SARS-CoV-2 lipoparticles incubated with MCF7 cells and potential SARS-CoV-2 Spike protein binding drugs. Results are shown as mean ±SD from at least 3 biological replicates, *p<0.05,**p<0.005, n.s.=not significant, unpaired two-tailed t-test.

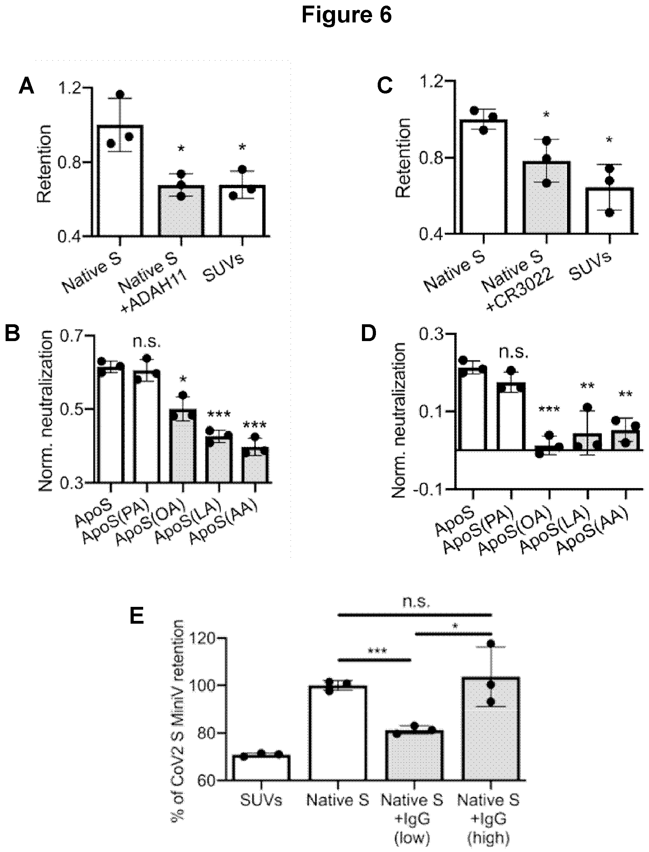

**Fig. 6:** (**A**) Retention assay for lipoparticles presenting native S and incubated with MCF7 cells for 24 h. Reduction in retention by ADAH11 nanobodies was measured by addition of 1 μM ADAH11 during the incubation period. (**B**) Native S-normalized neutralization of ADAH11 for lipoparticles presenting free fatty acid (FFA)-loaded apoS incubated with MCF7 cells for 24 hours. (**C**) Retention assay for lipoparticles presenting native S and incubated with MCF7 cells for 24 hours. Reduction in retention by CR3022 IgG was measured by addition of 132 nM CR3022 during the incubation period. (**D**) Native S-normalized neutralization of CR3022 based for lipoparticles presenting FFA-loaded apoS incubated with MCF7 cells for 24 h. (**E**) Retention analysis with native S-normalized neutralization of lipoparticles by convalescent COVID-19 patient serum-derived IgGs under low (0.1 μM) and high (1 μM) LA/AA concentrations. Results are shown as mean ±SD from at least 3 biological replicates, *p<0.05,**p<0.005, ***p<0.0005, unpaired two-tailed t-test.

## Examples

### Materials and Methods

*Materials*

18:1 DOPC 1,2-dioleoyl-sn-glycero-3-phosphocholesteroline, 18:1 DOPE 1,2-dioleoyl-sn-glycero-3-phosphoethanolamine, L-α-phosphatidylglycerol (EggPG), L-α-phosphatidylcholine (EggPC), 1,2-dioleoyl-sn-glycero-3-phosphoethanolamine-*N*-(lissamine rhodamine B Sulfonyl) (LissRhod PE /LissRhodamine PE), 18:1 DGS-NTA(Ni) 1,2-dioleoyl-sn-glycero-3-[(*N*-(5-amino-1-carboxypentyl)iminodiacetic acid)succinyl] (nickel salt), 18:1 1,2-dioleoyl-sn-glycero-3-phospho-(1'-myo-inositol) (ammonium salt), 18:1 1,2-di-(9Z-octadecenoyl)-sn-glycero-3-phospho-L-serine (sodium salt), 1,2-dioleoyl-sn-glycero-3-phosphoethanolamin Atto488-conjugate, 18:1 cholesterol, 18:0 N-stearoyl-D-erythro-sphingosylphosphorylcholine (SM), and extrude set with 50 nm pore size polycarbonate filter membranes were purchased from Avanti Polar Lipids, USA Dulbecco's Modified Eagle Medium (DMEM) high Glucose, heat inactivated fetal bovine serum, penicillin-streptomycin (10,000 U/mL), L-Glutamine (200 mM), trypsin-EDTA (0.05%), FluoroBrite DMEM, Cell-Tracker Green CMFDA dye, Hoechst 33342, phosphate buffered saline were purchased from Thermo Fischer Scientific, Germany. Dexamethasone (BioReagent), Tretinoin (pharmaceutical standard), Acitretin (>98%), Vitamin K2, Carbenoxolone disodium salt (>98%), palmitic acid (>99%), oleic acid (>99%), linoleic acid (>99%) arachidonic acid (analytical standard), oxalyl chloride (>99%), 3-picolylamine (>99%) were purchased from Sigma Aldrich, Germany. LinRGD (linear Arg-Gly-Asp tripeptide) was custom synthesized by PSL GmbH, Germany. Transparent flat bottom 96-well plates were purchased from TTP, Switzerland. Human Insulin was purchased from Millipore Sigma. Lipidex-1000 resin was purchased from Perkin Elmer, Germany. DMSO for cell culture use was purchased from Omnilab, Germany. Sicastar Ni2+-NTA silica beads were purchased from Micromod GmbH, Germany. Human IgG COVID-19 convalescent plasma fractionated purified and lyophilized was purchased from Innovative Research, USA. Anti SARS-CoV-2 S, clone CR3022 (FITC) was purchased from Novus Biologicals, Germany. Histidine-tagged recombinant SARS-CoV-2 spike RBD (Val16-Arg685(D614G)), human recombinant ACE2 were purchased from Sino Biologicals, Germany. MCF-7 cells were obtained from ATCC, USA. QCM-D sensor crystals (QS-QSX303) were obtained from Quantum Design GmbH, Germany.

*Lipoparticle and SUV preparation*

SUVs and lipoparticles were produced by manual extrusion through track-etched polycarbonate filter membranes. For this, lipids dissolved in chloroform stock solutions were mixed at the desired lipid ratio in glass vials and subsequently dried under vacuum for at least 15 min to evaporate the chloroform completely. The obtained lipid film was rehydrated to a final lipid concentration of 6 mM in PBS for at least 5 min and afterwards shaken for at least 5 min at 1000 rpm on a horizontal shaker. This liposome solution was extruded 9 times through a 50 nm radius pore size filter.
For immobilization of ectodomains, the SUV solution was diluted to a final concentration of 100 μM in PBS, corresponding to 10 μM final DGS-NTA(Ni$^{2+}$). To this, 5 μM of recombinant histidine-tagged ectodomain was added and incubated for coupling for at least 15 min. For loading of fatty acids (FAs) on lipoparticles, FAs were dissolved in DMSO to a final concentration of 100 mg/mL. From these stocks, 100 $\mu$g/mL dilutions in PBS were prepared. If not stated otherwise, from these dilutions, FAs were diluted to a final concentration of 3.3 $\mu$M into the lipoparticle samples.

*proteins expression and purification*

Recombinant wild-type (Wuhan) **SARS-CoV-2 S protein** with a mutated furin site was produced as described previously (Nature 581, 221-3 224 (2020)). The construct comprises amino acids 1 to 1213, lacks the native transmembrane domain which is replaced with a C-terminal thrombin cleavage site followed by a T4-foldon trimerization domain and a hexa-histidine tag. The polybasic cleavage site has been removed (RRAR to A mutation). Briefly, S protein was expressed using the MultiBac baculovirus expression system in Hi5 cells. Medium from transfected cells was harvested 3 days after-transfection by centrifuging the cultures at 1,000g for 10 min followed by another centrifugation of supernatant media at 5,000g for 30 min. This final supernatant was then incubated with 10 mL HisPur Ni-NTA Superflow Agarose (Thermo Fisher Scientific) per 4 litres of expression culture for 1h at 4 °C. Subsequently, the resin bound with SARS-CoV-2 S protein was collected using a gravity flow column (Bio-Rad) and then extensively washed with 30 column volumes (CV) of wash buffer (65 mM $NaH_2PO_4$, 300 mM NaCl, 20 mM imidazole, pH 7.5). Finally, protein was eluted using a step gradient of elution buffer (65 mM $NaH_2PO_4$, 300 mM NaCl, 235 mM imidazole, pH 7.5). After analysing elution fractions using reducing SDS-PAGE, fractions containing SARS-CoV-2 S protein were pooled and concentrated using 50 kDa MWCO Amicon centrifugal filter units (EMD Millipore) and finally buffer-exchanged in phosphate-buffered saline (PBS) pH 7.5. The protein was then subjected to size exclusion chromatography (SEC) using a Superdex 200 increase 10/300 column (GE Healthcare) in PBS pH 7.5. Peak fractions from SEC were then analysed using reducing SDS-PAGE and then fractions containing SARS-CoV-2 S protein were pooled and concentrated 5 using 50 kDa MWCO Amicon centrifugal filter units (EMD Millipore) and finally aliquoted and flash frozen in liquid nitrogen for storage at -80°C until further use.

The UK (or 'Kent') **B1.1.7 variant S** ectodomain gene sequence was synthesized and inserted into pACEBac1 plasmid (Genscript Inc., New Jersey USA). Expression and purification were carried out as described above for wild-type S.

The **S(R403A) mutant** was prepared by modifying the wild-type (Wuhan) S expression construct with the point mutation using the QuickChange site-directed mutagenesis kit (Qiagen).

**SARS-CoV** S encoding gene was synthesized (Genscript Inc, New Jersey USA). The construct comprises amino acids 14 to 1193, preceded by a GP64 secretion signal sequence (amino acids MVSAIVLYVLLAAAAHSAFA, SEQ ID No. 35) and contains a C-terminal thrombin cleavage site followed by a T4-foldon trimerization domain and a hexa-histidine affinity purification tag. The synthetic gene was inserted into pACEBac1 s. Protein was produced and purified as described above for SARS-CoV-2 S.

**MERS-CoV** S encoding gene was synthesized (Genscript Inc, New Jersey USA) and cloned into pACEBac1. This construct comprises amino acids 18 to 1294, preceded by the GP64 secretion signal sequence and contains a C-terminal thrombin cleavage site followed by a T4-foldon trimerization domain and a hexa-histidine affinity purification tag. Protein was produced and purified as described above for SARS-CoV-2 S.

**Apo SARS-CoV-2** S protein lacking free fatty acid was produced from purified SARS-CoV-2 S by Lipidex treatment. Briefly, purified SARS-CoV-2 S protein was incubated with pre-equilibrated lipidex-1000 resin (Perkin Elmer; cat no. 6008301) in PBS pH 7.5 overnight at 4°C on a roller shaker. Following this, Lipidex-treated S protein was separated from the resin using a gravity flow column. The integrity of the protein was confirmed by size exclusion chromatography (SEC) using a S200 10/300 increase column (GE Healthcare) and SDS-PAGE.

*ADAH11 selection, expression, and purification*

Neutralizing nanobody ADAH11 against the RBM of Spike ectodomains was selected from a synthetic library using *in vitro* selection by ribosome display. Following selection, the ADAH11 coding sequence was cloned into pHEN6 plasmid containing a PelB signal sequence at the N-terminus, and a hexa-histidine and 3X FLAG tag at the C-terminus. ADAH11 was expressed in *E. coli* TG1 cells in 2x YT medium overnight at 30°C induced with 1mM IPTG (Isopropyl $\beta$-D-1-thiogalactopyranoside). Cells were harvested by centrifugation at 3200g for 15 minutes at 4°C. Cells were then resuspend in 5 mL cold TES buffer (50 mM Tris pH 8.0, 20% Sucrose, 1 mM EDTA, complete protease inhibitor tablet) for each gram of pellet. This resuspension was incubated on a roller shaker for 45 minutes at 4°C. Then, 7.5 mL of ice-cold shock buffer (20 mM Tris pH 8.0, 5 mM $MgCl_2$) was added per gram of pellet and again incubated on a roller shaker for 45 minutes at 4°C. Then, the supernatant-containing periplasm was collected by centrifugation at 13,000g for 12 minutes at 4°C. This supernatant was incubated with 0.5 ml HisPur Ni-NTA Superflow Agarose (Thermo Fisher Scientific) per litre of expression for 1 hour. Resin was pre-equilibrated in ADAH11 wash buffer 1 (50 mM Tris, 200 mM NaCl, 10 mM Imidazole pH 8.0). A gravity flow column (Bio-Rad) was used to separate the resin bound with ADAH11 from unbound lysate. This resin was then washed with 20 CV of ADAH11 wash buffer 1, followed by 30 CV wash with ADAH11 wash buffer 2 (50 mM Tris pH 8.0, 200 mM NaCl, 20 mM Imidazole pH 8.0). Protein was then eluted using a step gradient of ADAH11 elution buffer (50 mM Tris, 200 mM NaCl, 500 mM Imidazole pH 8.0). Elution fractions were analyzed using reducing SDS-PAGE. Elution fractions containing ADAH11 protein were pooled and dialyzed against PBS pH 7.5. Dialyzed protein was concentrated using 10 kDa MWCO Amicon centrifugal filter units (EMD Millipore) and then injected on a Superdex 200 increase 10/300 size exclusion chromatography (SEC) column (GE Healthcare) in PBS pH 7.5. Peak fractions from SEC were analyzed using reducing SDS-PAGE and fractions containing ADAH11 were pooled and concentrated using 10 kDa MWCO Amicon centrifugal filter units (EMD Millipore). Finally, the protein was aliquoted and flash frozen in liquid nitrogen for storage at -80°C until further use.

*DLS + zeta potential*

Size and zeta potentials of SUVs and MiniV variants were measured with a Malvern Zetasizer Nano ZS system at a total lipid concentration of 100 $\mu$M in PBS. Temperature equilibration time was set to 300 s at 25 °C, followed by three repeat measurements for each sample at a scattering angle of 173° using the built-in automatic run-number selection. The material refractive index was set to 1.4233 and solvent properties to $\eta$ = 0.8882, n = 1.33 and $\varepsilon$ = 79.0.

*Hoechst staining and nuclei counting*

To assess blocking of cell adhesion under linRGD incubation, we imaged adherent cells after seeding and washing. To this end, MCF-7 cells were seeded at a density of 50.000 cells/well in flat-bottom 96-well plates in 100 $\mu$L culture medium. Cells were either incubated with 20 $\mu$M linRGD or with addition of 20 $\mu$L PBS (mock) for 24 hours. Subsequently, Hoechst33342 was added to a final concentration of 10 $\mu$M to the cell layers and incubated for 10 min. Cells were then washed twice with 200 $\mu$L PBS and fixed for 20 min with 4% paraformaldehyde. Cell nuclei were then imaged in the whole well with a Leica DMi8 inverted fluorescent microscope equipped with an sCMOS camera and 10x HC PL Fluotar (NA 0.32, PH1) objective with DAPI emission/excitation filters. For automated nuclei counting, TIFF images from 3 wells (i.e. three replicates) were background segmented by global histogram thresholding and automated particle counting (particle analysis) with ImageJ software (NIH). Before particle counting, a watershed algorithm was applied to separated overlapping nuclei and nuclei counting was restricted to particles in the size range between 1 $\mu$m$^2$ and 100 $\mu$m$^2$.

*Cryo-TEM tomography*

For cryo-TEM imaging, lipoparticles were diluted to a final particle concentration of $2*10^2$ particles/mL. The samples were applied to glow-discharged C-Flat 1.2/1.3 4 C grids (Protochips) and plunge-frozen using a Vitrobot Mark IV (FEI, now Thermo Scientific) at the following settings: temperature of 22 °C, humidity of 100%, 0s wait time, 2-3s blot time, blot force -1, and 0s drain time. The data were acquired on a Titan Krios electron microscope operated at 300 kV equipped with a Falcon 3EC direct detector, a Volta phase plate, and a Cs Corrector (CEOS GmbH). The micrographs were acquired with the software EPU (Thermo Scientific) in linear and counting mode at magnified pixel sizes of 1.39 Å and 0.85 Å, at doses of 40-60 e-/sqÅ, at defocus values ranging from -0.25$\mu$m to -2.0 $\mu$m using the Volta phase plate or the 100 $\mu$m objective aperture. Tilt series were acquired with the software TOMO (Thermo Scientific) at tilt angles ranging from -60° to 60° at 3° increments with defocus values ranging from -0.3 $\mu$m to -1.0 $\mu$m with individual micrograph movies recorded with 10 frames in counting mode at a magnified pixel size of 0.85 Å, a total dose per micrograph of 3e-/sqÅ, using the Volta phase plate. Micrograph movies were aligned using MotionCor2 in the RELION3 software suite. Tilt series were binned by a factor of four for further processing in Etomo (IMOD). For each tilt series, the micrographs were aligned using patch tracking. The final tomograms were generated with a filtered back-projection of the aligned micrographs. Tomogram slices were FFT band-pass filtered between 3 and 40 pixel and subsequently a 2x2 pixel Gaussian blur filter was applied. Images were contrast corrected by visual inspection.

*QCM-D + imaging SLB*

For QCM-D measurements, sensor crystals, AT-cut gold electrodes coated with a 50 nm thick layer of silicon oxide were used. SiO$_2$ surfaces were cleaned as described elsewhere. Briefly, QCM-D sensor crystal were cleaned using an aqueous 2 % SDS solution water and activated by UV/ozone for 10 min. A QSense Analyzer equipped with a four channel system from QuantumDesign was used for measurements. All measurements were performed at 22 °C in an open mode. Resonance frequency and dissipation shifts were recorded at several harmonics simultaneously. Before adding the samples, the frequency and dissipation changes were base-lined by averaging over the last 5 min of the buffer wash (PBS). Formation of supported lipid bilayers (SLBs) was achieved by absorption and rupture of SUVs on cleaned SiO$_2$ surfaces. SUVs with a lipid composition of 20 mol% L-$\alpha$-phosphatidylglycerol (EggPG), 77 mol% L-$\alpha$-

phosphatidylcholine (EggPC), 1,2-Dioleoyl-sn-glycero-3-phosphoethanolamin, 2 mol% 1,2-dioleoyl-sn-glycero-3-[(N-(5-amino-1-carboxypentyl)iminodiacetic acid)succinyl] (18:1 DGS-NTA) and 1 mol% 1,2-Dioleoyl-sn-glycero- 3-phosphoethanolamin Atto488-conjugate were used for SLB formation at a lipid concentration of 1.2 mM and a final $MgCl_2$ concentration of 2mM. 200 µL of this solution was added to each QCM-D sensor to form SLBs after the sensors were equilibrated with 200 µL PBS for 7 min and incubated for 17 min. SLB formation was confirmed by the characteristic changes in frequency and dissipation as it is described elsewhere. SLBs were washed with PBS to remove non-ruptured SUVs for 7 min. For immobilization of recombinant human ACE2 200 µL PBS containing 300 nM histidine-tagged ACE2 was added to the according sensor crystals (S1, S2 and S4) and incubated 5 for 16 min. Unbounded ACE2-receptors were removed washing with PBS. The crystal sensor S3 not being functionalized with the ACE2-receptor was treated in the same way with the washing buffer . After 11 min, the washing buffer was removed and 200 µL of a solution containing 1.5 µM MiniVs (total lipid concentration) was added to sensors S1 and S3. To the crystal of sensor S2, a 200 µL PBS solution containing 1.5 µM (total lipid concentration) naive SUVs was added. Sensor S4 was treated with 200 µL PBS containing soluble recombinant S. The solutions were incubated on the crystal sensors for 55 min and afterwards washed with PBS to remove unbound components.

*Cell culture and viruses*

MCF-7 cells were cultured in Dulbecco's Modified Eagle Medium supplemented with 4.5 g/l glucose, 1 % L-glutamine, 1 % penicillin/streptomycin, 0.01 mg/mL recombinant human insulin and 10 % fetal bovine serum. Cells were routinely cultured at 37 °C and 5 % $CO_2$ atmosphere and passaged at ~80 % confluency by detachment with 0.05% trypsin/EDTA treatment. MCF-7 cells were cultured in Dulbecco's Modified Eagle Medium (DMEM) supplemented with 4.5 g/l glucose, 1 % L-glutamine, 1 % penicillin/streptomycin, 0.01 mg/mL recombinant human insulin and 10 % fetal bovine serum. A549-ACE2 cells were kindly provided by M. Cortese and R. Bartenschlager (Heidelberg University) and cultivated in DMEM supplemented with 10% fetal calf serum (Capricorn), 100 U/mL penicillin, 100 µg/mL streptomycin and 1% non-essential amino acids (all from Gibco) and 0.5 mg/mL G418. Cells were routinely cultured at 37 °C and 5 % $CO_2$ atmosphere and passaged at ~80 % confluency based on 0.05 % trypsin/EDTA treatment.

The SARS-CoV-2 isolate Bavpat1/2020 was obtained by the European Virology Archive (Ref-SKU: 026V-03883) at passage 2. Virus stocks were generated by passaging the virus two times in VeroE6 cells. Virus stocks were titrated by infection of VeroE6 cells.

*Confocal microscopy*

Confocal microscopy was performed with a laser-scanning microscope LSM 800 (Carl Zeiss AG). Images were acquired with a 20x (Objective Plan-Apochromat 20x/0.8 M27, Carl Zeiss AG) and a 63x immersion oil objective (Plan-Apochromat 63x/1.40 Oil DIC, Carl Zeiss AG). Images were analyzed with ImageJ (NIH) and adjustments to image brightness and contrast as well as background corrections were always performed on the whole image and special care was taken not to obscure or eliminate any information from the original image.

*Mass Spectrometry*

For loading of Apo S samples with defined FFA for MS analysis, pure FFA (PA, OA, LA, AA) solutions were prepared at a stock concentration of 100 mg/mL in DMSO. Then, a prediluion of 100 µg/mL in PBS was prepared and 0.2 mg Apo S samples were mixed with respective FFA predilutions in a 1:10 molar ratio and incubated for 2 h. Subsequently, the histidine tagged Apo protein was pulled down with 1 mg of 300 nm NTA($Ni^{2+}$)-conjugated silica beads through 30 min incubation and subsequent centrifugation at 11,000 g for 5 min. Apo S was eluted by addition of 100 mM imidazole and subsequently subjected to MS samples preparation (see below). For mass spectrometry detection of the FA content in S samples, a targeted LC-coupled MS/MS approach with multiple reaction monitoring was applied. For MS analysis, all protein samples were pre-diluted to a final concentration of 400 µg/ mL. To extract the FAs from the S protein, S samples were mixed in a 1:4 (v/v) ratio with chloroform for 2 hours on a horizontal shaker in a Teflon sealed glass vial at 25°C. The top organic phase was then transferred to a new glass vial and the chloroform was evaporated for 15 min in a desiccator. Subsequently, a derivatization approach enabling for improved MS detection of FFAs was applied. This approach is based on the activation of carboxylic FA head groups with oxalyl chloride (OC) and subsequent derivatization with 3-picolylamine. For this, 200 µL of a 2M OC solution prepared in dichloromethane (DCM) was added to the samples using a glass syringe and incubated for 5 min at 65°C. The OC and DCM were then removed in a desiccator and 150 µL of a 3-picolylamine solution (1% (v/v) in acetonitrile) were added to the samples and incubated for 5 min. The residual solvent was again evaporated in a desiccator and the samples were dissolved in 50 µL acetonitrile. To all samples, a deuterated LA internal standard (dissolved in DCM) was added to a final concentration of 20 µg/mL before derivatization. Finally, the resulting samples were diluted 1 to 500 in 80% ACN aq. (MS grade).

The subsequent LC-MSMS analysis was performed using a Shimadzu Nexera system hyphenated to a Sciex QTRAP 4500 system. The LC system was supplied with water (A) and acetonitrile (B) in LCMS grade from Biosolve. The solvents were supplemented with 0.1 % LCMS grade formic acid. 6 $\mu$L of the sample solutions were fractionated with a Supelco Titan C18 column 100 x 2.1 mm, 1.9 $\mu$, at 45°C. Whereas the following gradient was applied: 0 min -40% B; 1.5 min: 40% B; 6.5 min - 98% B; 8.0 min - 98% B; 8.1 min -40% B and 9.5 min - 98% B. The mass spectrometer was controlled using the Analyst 1.7 software and was operated in ESI positive mode. An optimal target compound ionization was achieved by setting the following source parameters: Curtain gas 35, temperature 550 °C, ionization voltage 5500 V, nebulizer gas 65, heater gas 80 and collision gas 9.

The targeted derivatized fatty acids were detected in MRM mode. Fragmentation of the monitored PA derivatized FA yielded a characteristic MSMS fragment with m/z 92. For linoleic acid additional parent to fragment MSMS transitions were recorded. The following compound dependent MSMS parameters were applied: PA derivatized linoleic acid (LAP-92: 371.3 - 92.1 Da, Dwell 90 ms, declustering potential (DP) 80, entrance potential (EP) 10, collision energy (CE) 43 , cell exit potential (CXP) 11, LAP-109: 371.3 - 109.1 Da, Dwell 40 ms, DP 80, EP 10, CE 36, CXP 10, LAP-163: 371.3 - 163.1 Da, Dwell 15 ms, DP 80, EP 10, CE45, CXP 11); PA derivatized deuterated linoleic acid (LADP-92: 375.3 - 92.1 Da, Dwell 50 ms, DP 80, EP 10, CE 43, CXP 11); PA derivatized palmitic acid (PAP-92: 347.3 - 92.1 Da, Dwell 50 ms, DP 80, EP 10, CE 43, CXP 11); PA derivatized oleic acid (OAP-92: 373.3 - 92.1 Da, Dwell 50 ms, DP 80, EP 10, CE 43, CXP 11); PA derivatized arachidonic acid (AAP-92: 395.3 - 92.1 Da, Dwell 50 ms, DP 80, EP 10, CE 43, CXP 11). Subsequent data analysis was performed using the Analyst and Multiquant 3.0.2 software.

A set of positive and negative control samples was measured under the same conditions to ensure high quality results. As positive controls derivatized LA standard dilutions in 80% ACN aq. (MS grade) starting from the following concentration levels were used: 5, 10 and 25 $\mu$g/mL. 4-fold injection of the 25 $\mu$g/mL LAP standard yielded a CV <2%. A sample preparation related contamination of the samples was exclude using a negative control sample, that was generated by using a water aliquot instead of the protein solution for the sample work-up. Moreover, we ensured 30 the chromatographic separation of LADP ($C_{18}H_{28}D_4O_2$) and the ubiquitous as well as isobaric stearic acid ($C_{18}H_{36}O_2$).

*Retention assays*

For quantification of lipoparticle-cell binding, retention assays were developed to measure the amount of lipoparticles retained within culture plates after incubation and washing. This assay is based on quantification of the rhodamine fluorescence form the SUV lipid membrane and can therefore not discriminate between attachment and uptake of lipoparticles. For retention analysis, SUVs and lipoparticles with different recombinant Spike ectodomains (as indicated in the figure legends), were added to MFC-7 cell cultures in flat-bottom 96 well plates with 100 $\mu$L culture medium to a final lipid concentration of 10 $\mu$M. Before addition, cell medium was exchanged from the seeding medium (DMEM 40 supplemented with phenol red, 4.5 g/l glucose, 1 % L-glutamine, 1 % penicillin/streptomycin, 0.01 mg/mL recombinant human insulin and 10 % fetal bovine serum) to low-serum medium (DMEM supplemented without phenol red, 4.5 g/l glucose, 1 % L-glutamine, 1 % penicillin/streptomycin, 0.01 mg/mL recombinant human insulin and 0.5 % fetal bovine serum) to reduce the amount of serum-derived FAs far below physiologically relevant concentrations. After incubation of lipoparticles with cells for 24 hours, rhodamine fluorescence was measured at 9 different positions and 1300 $\mu$m distance to well wall in each well using an Infinite M200 TECAN plate reader controlled by TECAN iControl software with an in-built gain optimization and excitation/emission setting adjusted to 555/585 nm. Wells were then washed three times with PBS and subsequently fixed with 100 $\mu$L 4% paraformaldehyde. After 10 min fixation, rhodamine fluorescence was again measured in each well with the settings mentioned above. For retention analysis binding could then be deduced from residual fluorescence in each well (for assessment of patient IgG neutralization) or retention values could be calculated by dividing the residuals fluorescence to the initial fluorescence intensity before washing. All measurements were performed in triplicates. As retention show significant variations depending on cell seeding density, cell viability and vesicle preparation, a condition of naive SUVS (i.e. without protein on the surface) was added to all experimental batches for normalization proposes. For time resolved measurements of retention, separate wells for each time point were prepared and evaluated 5 sequentially.

For retention assay involving an assessment of FFA influence, stock of pure FFA (PA, OA, LA and AA) were prepared in DMSO at a final concentration of 100 mg/mL. From these stock, pre dilutions (100 $\mu$g/mL) in PBS were prepared. Individual FFAs, as indicated in the figures, were added to the culture medium of the retention assays at a final concentration of 1 $\mu$M.

*Competition experiments of SARS-CoV-2 D614G and SARS-CoV-2 B1.1.7 S*

For assessment of competitive cell binding between lipoparticles presenting WT(D614G) and B1.1.7 S on the surface, two differently fluorescent SUV samples were prepared. SUVs harbouring Atto448 we decorated with WT(D614G) S and SUVs harbouring rhodamine B with B1.1.7 S. 10 $\mu$M final lipid concentration of both lipoparticle types were incubated together with MCF-7 cells and retention assays were performed as described above for the rhodamine and Atto488 (emission excitation set to 488/512) signals separately. Naive SUV controls were performed for both vesicle types and retention were normalized accordingly. Based on the differing fluorescence of the two lipoparticle populations, multiplicity of infection (MOI) measurements were performed by confocal imaging. For this, both lipoparticle types (D614G and B1.1.7) were incubated with MCF-7 cell in glass bottom 8-well LabTek chambers for 24 hours. After incubations, cells were washed three times with PBS and subsequently imaging in at least 8 z-positions (1 $\mu$m stack distance) in both channels. Maximum intensity projects were made by ImageJ and lipoparticles of each type were manually counted for 5 individual cell groups.

*Retention assays (RGD)*

For assessment of RGD-motif-mediated effects in lipoparticle cell binding, retention assay under integrin blocking conditions with linRGD were performed. For this, 100.000 MCF-7 cells/ well were seeded in flat bottom 96-well plates and allowed to form confluent monolayers overnight. Subsequently, seeding medium was exchanged to lowserum (0.5%) cell culture medium and a final linRGD concentration of 20 $\mu$M was added to each well from a 10 mM stock solution. Lipoparticles (with S-configurations as indicated in the figure legends) were added to a final lipid concentration of 10 $\mu$M. Retention assays were performed as detailed above.

To quantify differences in FA-regulated S integrin engagement, retention was calculated as linRGDnormalized retention. For this, retention of lipoparticles was measured with and without incubation of linRGD. Differences in retention were calculated and normalized by:

$$\frac{R - R_{linRGD}}{R_{nativeS}}$$

Where R is the retention value of the respective lipoparticle S configuration without linRGD, $R_{linRGD}$ is the retention value of the corresponding lipoparticle S configuration under addition of 20 $\mu$M linRGD and $R_{nativeS}$ is the retention value lipoparticles presenting native S.

*Retention assays (FABP drug assessment)*

For assessment of drug-modulated Spike protein binding of potential pharmacologic FABP binders, retention assays under drug incubation were performed. For this, 100,000 MCF-7 cells/ well were seeded in flat bottom 96-well plates and allowed to form confluent monolayers overnight. Subsequently, seeding medium was exchanged to low-serum (0.5%) cell culture medium and a final drug concentration of 1 $\mu$M was added to each well from DMSO stock solutions. Lipoparticles (with S-configurations as indicated in the figure legends) were added to a final lipid concentration of 10 $\mu$M and incubated for 24 hours. For normalization purposes, also retention of naive SUVs under drug treatment were measured to account for any drug-induced changes in cellular phenotypes. Retention assays were performed as detailed above and a drug-normalized retention was calculated from:

$$\frac{R \times R_{corr}}{R_{apo}}$$

Where $R_{corr}$ is calculated by

$$\frac{R_{SUVs}}{R_{drugSUVs}}$$

and R is the retention value of the respective lipoparticle Apo S configuration under drug incubation, $R_{apo}$ is the retention value of lipoparticles with Apo S without drug incubation, $R_{SUVs}$ is the retention value of SUVs without drug incubation and $R_{drugSUVs}$ is the retention value of SUVs under drug incubation.

*Protein structure visualization and ASA calculation*

Previous studies have demonstrated the value of accessible surface area calculations for assessment of IgG epitope characterization in SARS-CoV-2 S and conformational changes upon protein binding. For visualization of Spike cryoTEM structures, the PDB 3D viewer was applied. ASAs were calculated from molecular surface representations of residue accessible surface area properties in the PBD. A rolling probe radius of 1.4 nm was applied. For ASA calculations, hydrogen atoms were taken into consideration. Tracing atoms were not considered for the analysis and no line size attenuation was applied.

*Nanoparticle tracking analysis*

Lipoparticle concentration was determined by NTA with a ZetaView Quatt Video Microscope PMX-420 (Particle Metrix, Inning am Ammersee, Germany). Alignment was performed using 100 nm polystyrene beads diluted 1:250000 (v:v) in MilliQ water. Lipoparticles were diluted in PBS to a final concentration of 50-150 particles/frame. The observation cell was equilibrated with PBS before 1 mL of the sample was injected. One acquisition cycle was performed at 11 positions of the observation cell, in scatter mode using the 488 nm laser and at a temperature of 24 °C. The following settings were used for acquisition: sensitivity 80, shutter 100, frame rate 30, medium quality. Postacquisition parameters were set as follows: minimal brightness 30, minimal area 10, maximal area 1000, trace length 15. All 11 positions were included in the analysis, using over 500 traced particles in total.

*Lipoparticle neutralization assays*

For lipoparticle neutralization assays, 100,000 MCF-7 cells/ well were seeded in flat bottom 96-well plates and allowed to form confluent monolayers overnight. Subsequently, seeding medium was exchanged to low-serum (0.5%) cell culture medium and lipoparticles (with S configurations as detailed in the figure legends) were added to a final lipid concentration of 10 $\mu$M. Three types of neutralizing immunoglobulins were tested 1. IgG CR3022 2. ADAH11 and 3. purified IgG from convalescent COVID-19 donors. CR3022 neutralization was assessed by addition of 132 nM purified IgG. ADAH11 neutralization was titrated in a concentration range of 7.4 nM and 1.5 $\mu$M and retention assays for ADAH11 neutralization were performed at 1 $\mu$M ADAH11. Neutralization of purified donor IgGs was measured at a final concentration of 3.3 $\mu$g/mL.

For assessment of neutralization with different FFA profiles, immunoglobulin mediated reduction in retention was calculated by:

$$\frac{R - R_{immunoglobulin}}{R_{nativeS}}$$

where R is the retention value of the respective lipoparticle Apo S configuration without immunoglobulin, $R_{immunoglobulin}$ is the retention value of the respective lipoparticle Apo S configuration under immunoglobulin incubation and $R_{nativeS}$ is the retention value of lipoparticles presenting native S without immunoglobulin incubation. For the assessment of lipoparticle neutralization as a function of FFAs, individual FFAs were added from DMSO stocks (100 mg/mL) to the culture medium of the retention assay at a final concentration of 1 $\mu$M. To mimic the basal, low FFA levels, for COVID-19 donor IgG neutralization, no additional FFAs were added to the culture medium as the 0.5% serum concentration of the retention assay culture medium already provide approximately 0.1 $\mu$M LA and AA.

*Competition assays*

5x10$^4$ A549-ACE2 cells were seeded in 24-well plates on the day prior to infection. Cells were incubated with 300 $\mu$L of SUV or lipoparticle dilution for 2.5 h. 100 $\mu$L of virus suspension containing 5x10$^4$ infectious virus particles (multiplicity of infection of 1) were added to each well and incubated for 2 h. Medium was removed, and cells washed two times with sterile PBS before addition of 1 mL of fresh medium. Cells were harvested 18 h post infection and total RNA extracted using NucleoSpin RNA Plus kit (Macherey-Nagel), following manufacturer's instructions. cDNA was generated using the High-Capacity cDNA Reverse Transcription kit (Applied Biosystems) following manufacturer's instructions. Expression levels of GAPDH and SARS-CoV-2 Orf7a mRNA were determined by using the iTaq Universal SYBR Green 2x (Bio-Rad). Reactions were performed on an CFX96 (Bio-Rad) using the following program: 95 °C for 3 min and 45 cycles as follows: 95 °C for 10 s, 60 °C for 30 s. GAPDH mRNA level was used for normalization of input RNA. Relative abundance of each specific mRNA was determined by using the $\Delta\Delta$CT method. The following primers were used:

GAPDH-For 5' - GAAGGTGAAGGTCGGAGTC - 3'
GAPDH-Rev 5' - GAAGATGGTGATGGGATTTC - 3'
CoV-2 Leader_For 5' - TCCCAGGTAACAAACCAACCAACT- 3'
CoV-2 Orf7a_Rev 5' - AAATGGTGAATTGCCCTCGT- 3'.

**Example 1:** Lipoparticles with recombinant Spike protein ectodomains

Lipoparticles were produced as described above from a lipid formulation composed of 45 mol% DOPC, 21 mol% DOPE, 3 mol% DOPS, 12 mol% DOPI, 14 mol% cholesterol, and 3 mol% N-stearoyl-D-erythro-sphingosylphosphorylcholine (SM), and decorated with recombinant SARS-CoV-2 D614G S ectodomains (C1-G1236 with C-terminal trimerization domain and histidine-tag), or recombinant B1.1.7 variant S ectodomain, or recombinant SARS-CoV-2 R403A S ectodomains, or recombinant SARS-CoV S ectodomains, or recombinant MERS-CoV S ectodomains, or recombinant apo SARS-CoV-2 D614G S ectodomains, each *via* NTA(Ni$^{2+}$) functionalized 18:1 DGS. A negative zeta potential of -39.2 mV in $H_2O$ and -3.4 mV in isosmotic buffer were measured, which is consistent with natural SARS-CoV-2 virions. S ectodomain immobilization and lipoparticle ultrastructure was verified by cryo-EM tomographic imaging (Figure 2). On average, 14.6 ($\pm$5.4, n=5 vesicles) spikes per vesicle were counted, which is comparable to the trimer density found on live SARS-CoV-2 virions. These immobilized, prefusion-stabilized S variants with deleted polybasic cleavage site were applied to specifically analyze RBD-mediated receptor binding independently from S2-induced membrane fusion. Lipoparticle functionality and binding specificity towards ACE2 receptors was verified by quartz crystal microbalance with dissipation monitoring (QCM-D). It was found that lipoparticles specifically bind to ACE2 receptors with neglectable non-specific attachment to lipid membranes (see Figure 3). Taken together, the inventive lipoparticles accurately mimic the structure and receptor binding of natural virions.

**Example 2:** Retention assay

Hepatitis B virus lipoparticles were prepared as follows: small unilamellar vesicles with a lipid composition of 83 mol% DOPC, 7 mol% DOPE, 5 mol% DOPI, 3 mol% SM, 1 mol% DGS-NTA and 1 mol% LissRhodamine PE were produced by mechanical extrusion in the same way the minimal SARS-CoV-2 lipoparticles described above. Retention of this liposomes to HepG2 cells was either measured as plain lipoparticles (Figure 4, B) or as lipoparticles that were conjugated with recombinant Hepatitis B surface Antigen adw subtype 2 proteins (mutated by replacing the Methionine residue at position 133) via a histidine-tag on the C-terminus of the protein (Figure 4, E).

Dengue virus lipoparticles were prepared as follows: small unilamellar vesicles with a lipid composition of 40 mol% DOPC, 30 mol% DOPE, 22 mol% SM, 5 mol% ceramides, 1 mol% DOPS, 1 mol% DGS-NTA and 1 mol% LissRhodamine PE were produced my mechanical extrusion in the same way the minimal SARS-CoV-2 lipoparticles described above. Retention of these lipoparticles to HepG2 cells was either measured as plain lipoparticles (Figure 4, C) or as lipoparticles that were conjugated with recombinant Dengue virus 2 E-glycoprotein (SEQ ID No. 30) via a histidine-tag on the C-terminus of the protein (Figure 4, F).

**Example 3:** Influence of the fatty acid binding pocket (FABP) on regulation of SARS-CoV-2 spike protein cell binding

Lipoparticles allowed for a systematic assessment of changes in SARS-CoV-2 spike protein (S)-mediated cell interactions as a function of FABP occupancy. Because recombinant native S binds an *a priori* undefined set of free fatty acids (FFAs) during expression and purification, first FFA-depleted S (apoS) was generated by treatment of the purified native S samples with alkoxylated lipophilic columns. Compared to the native S, apoS-decorated lipoparticles displayed increased binding to human epithelial cells **(Figure 5A)**. This is consistent with a model where FFAs lock a closed RBD conformation, thereby reducing exposure of the receptor binding motif (RBM). Then controlled loading of apoS lipoparticles was performed by incubating them with FFAs of different lengths and saturation (i.e., palmitic acid (PA), oleic acid (OA), linoleic acid (LA) and arachidonic acid (AA)) that showed most prominent changes in lipidomic profiles during COVID-19 infection. Binding of OA, LA and AA, but not PA, in S was successfully verified by multiple reaction monitoring LC-MS/MS. Of note, addition of saturated PA did not significantly reduce lipoparticle-cell binding compared to apoS. However, it was observed that the (poly)-unsaturated FFAs OA, LA and AA were able to reduce lipoparticle binding compared to apoS lipoparticle **(Figure 5A)**.

It was further observed that S(R403A)-presenting lipoparticles (without RGD motif) exhibited significantly reduced cell binding compared to native S lipoparticles **(Figure 5B)**. This suggests that the RGD motif directly contributes to S-based cell binding.

It was further investigated if the FABP also regulates S-integrin binding, by measuring changes in the binding efficiency of FFA-loaded apoS lipoparticles blocked with linRGD **(Figure 5C)**. It was found that unsaturated FFAs influence the engagement of integrins by S, most likely by modulation of the open-to-close RBD equilibrium. Taken together, this demonstrates that the FABP is also able to regulate RGD exposure and enhance integrin engagement by SARS-CoV-2 S.

In the search for therapeutic FABP antagonists, drug-normalized retention of apoS lipoparticles were measured during treatment with five potential antagonists (Figure 5D). Two drugs, vitamin K and dexamethasone were found that reduce S-mediated binding of the lipoparticles in a FABP-regulated manner. This demonstrates that the FABP is a potentially druggable regulator of SARS-CoV-2 cell binding.

**Example 4:** Regulation of S immunogenicity against neutralizing immunoglobulins by FABP

In order to study the influence of the FABP on S immunogenicity a VHH nanobody against the receptor binding motif (ADAH11) was raised *in vitro* using ribosome display. ADAH11 efficiently reduced the binding of native S lipoparticles (Figure 6A) with an $ED_{50}$ of 117 nM. Importantly, ADAH11 neutralization was strongly dependent on S loading with free fatty acids, where again only unsaturated FFAs reduced the neutralization efficiency. This is in agreement with reduced neutralization for closed RBD states (Figure 6B). In line with this, it was found that CR3022 (open RBD only binder) is likewise able to reduce cell binding of native S lipoparticles (Figure 6C) and CR3022 neutralization activity was sensitive to S loading with unsaturated FFAs (Figure 6D). Taken together, this demonstrates that FFA-binding can regulate S neutralization by IgGs by reducing exposure of NIDS.

It was further observed that patient serum IgGs effectively neutralize lipoparticle binding under basal LA/AA concentration but loses neutralization activity under elevated LA/FA conditions (Figure 6E). This suggests that FFA binding by S modulates the exposure of immunodominant sites, thereby coupling FFA concentrations to S immunogenicity.

**Sequence Listing**

| SEQ ID NO | Description |
|---|---|
| 1 | SARS-CoV-2 Signal peptide, S1 Subunit, Receptor Binding Site, S2 Subunit |
| 2 | native SARS-CoV-2 Spike ectodomain protein construct, furin cleavage site RRAR replaced by A, C-terminal thrombin cleavage site, T4 foldon trimerization domain, hexa-His tag |
| 3 | C-terminal thrombin cleavage site |
| 4 | T4 foldon trimerization domain |
| 5 | SARS-CoV-2 Spike protein native ectodomain |
| 6 | SARS-CoV-2 Spike protein native ectodomain L4F mutant |
| 7 | SARS-CoV-2 Spike protein native ectodomain T6N mutant |
| 8 | SARS-CoV-2 Spike protein native ectodomain P12S mutant |
| 9 | SARS-CoV-2 Spike protein native ectodomain D66A mutant |
| 10 | SARS-CoV-2 Spike protein native ectodomain D124Y mutant |
| 11 | SARS-CoV-2 Spike protein native ectodomain R176S mutant |
| 12 | SARS-CoV-2 Spike protein native ectodomain D201G mutant |
| 13 | SARS-CoV-2 Spike protein native ectodomain R232I mutant |
| 14 | SARS-CoV-2 Spike protein native ectodomain K403N mutant |
| 15 | SARS-CoV-2 Spike protein native ectodomain K403T mutant |
| 16 | SARS-CoV-2 Spike protein native ectodomain L411R mutant |
| 17 | SARS-CoV-2 Spike protein native ectodomain E470K mutant |
| 18 | SARS-CoV-2 Spike protein native ectodomain N487Y mutant |
| 19 | SARS-CoV-2 Spike protein native ectodomain A556D mutant |
| 20 | SARS-CoV-2 Spike protein native ectodomain D600G mutant |
| 21 | SARS-CoV-2 Spike protein native ectodomain H641Y mutant |
| 22 | SARS-CoV-2 Spike protein native ectodomain Q663H mutant |
| 23 | SARS-CoV-2 Spike protein native ectodomain Q663P mutant |
| 24 | SARS-CoV-2 Spike protein native ectodomain P667H mutant |
| 25 | SARS-CoV-2 Spike protein native ectodomain A684V mutant |
| 26 | SARS-CoV-2 Spike protein native ectodomain T699I mutant |
| 27 | SARS-CoV-2 Spike protein native ectodomain S965A mutant |
| 28 | SARS-CoV-2 Spike protein native ectodomain T1010I mutant |

(continued)

| SEQ ID NO | Description |
|---|---|
| 29 | SARS-CoV-2 Spike protein native ectodomain D1101H mutant |
| 30 | Recombinant Dengue virus 2 E-glycoprotein (His tag) |
| 31 | GAPDH-For 5' primer |
| 32 | GAPDH-Rev 5' primer |
| 33 | CoV-2 Leader_For 5' primer |
| 34 | CoV-2 Orf7a_Rev 5' primer |
| 35 | GP64 secretion signal sequence |

**Claims**

1. A non-infectious synthetic lipoparticle comprising:

   a lipid bilayer comprising

   - between 40 and 85 mol percent of a phosphatidylcholine as neutral amphiphile,
   - at least one anionic lipid, and
   - at least 1 mol percent of a lipid-bound Spike protein ectodomain, wherein the Spike protein ectodomain binds to a cell surface receptor,

   wherein the lipoparticle has a diameter between 20 nm and 300 nm as measured by dynamic light scattering.

2. The lipoparticle according to claim 1, wherein the Spike protein ectodomain is selected from the SARS-CoV-2 Spike protein ectodomain or a mutant thereof, SARS-CoV Spike protein ectodomain or a mutant thereof, MERS-CoV Spike protein ectodomain or a mutant thereof, human immunodeficiency virus type 1 envelope Spike protein ectodomain or a mutant thereof, large Hepatitis B virus envelope Spike protein ectodomain or a mutant thereof, Hepatitis C virus or a mutant thereof, Herpes simplex virus 1 glycoprotein B, C, D, H, or L ectodomain or a mutant thereof, Herpes simplex virus 2 glycoprotein B, C, D, H, or L ectodomain or a mutant thereof, Ebolavirus glycoprotein ectodomain or a mutant thereof, Marburg virus glycoprotein ectodomain or a mutant thereof, Influenza virus A hemagglutinin ectodomain or mutant thereof, Influenza virus B hemagglutinin ectodomain or mutant thereof, Influenza virus C hemagglutinin ectodomain or mutant thereof, Influenza virus D hemagglutinin ectodomain or mutant thereof, Dengue virus E envelope protein ectodomain or a mutant thereof, and Zika virus Spike protein ectodomain or a mutant thereof.

3. The lipoparticle according to claim 1 or 2, wherein the Spike protein ectodomain is a SARS-CoV-2 Spike protein ectodomain mutant homotrimer, wherein each monomer of the SARS-CoV-2 Spike protein ectodomain comprises an amino acid sequence identical to the sequence as set forth in SEQ ID NO 5 and wherein each monomer comprises further a single point mutation at one of the following sites: L4, T6, P12, D66, D124, R176, D201, R232, K403, L411, E470, N487, A556, D600, H641, Q663, P667, A684, T699, S965, T1010, and D1101.

4. The lipoparticle according to any one of claims 1 to 3, wherein the Spike protein ectodomain is a SARS-CoV-2 Spike protein ectodomain mutant homotrimer, wherein each monomer comprises an amino acid sequence identical to one of the sequences as set forth in SEQ ID NOs 6 to 29.

5. The lipoparticle according to any one of claims 1 to 4, wherein the Spike protein ectodomain is an apo Spike protein ectodomain devoid of free fatty acids.

6. The lipoparticle according to any one of claims 1 to 5, wherein the lipid bilayer further comprises at least one cationic lipid.

7. The lipoparticle according to any one of claims 1 to 5, wherein the lipid bilayer comprises

   40-50 mol%    1,2-dioleoyl-sn-glycero-3-phosphocholine,

(continued)

| | |
|---|---|
| 15-25 mol% | 1,2-dioleoyl-sn-glycero-3-phosphoethanolamine, |
| 2-4 mol% | 1,2-dioleoyl-sn-glycero-3-phospho-l-serine, |
| 12 mol% | 1,2-dioleoyl-sn-glycero-3-phospho-(1'-myo-inositol), |
| 14 mol% | cholesterol, and |
| 3 mol% | $N$-stearoyl-D-erythro-sphingosylphosphorylcholine. |

8. The lipoparticle according to any one of claims 1 to 7, wherein the Spike protein ectodomain is bound via a His-tag to a Ni-NTA functionalized lipid.

9. The lipoparticle according to any one of claims 1 to 8, wherein the cell surface receptor is selected from angiotensin-converting enzyme 2, DPP4, CD4, CCR5, NTCP, CD81, SR-BI, DC-SIGN, L-SIGN, heparan sulphate proteoglycans, asialoglycoprotein receptors, herpesvirus entry mediator, nectin-1, nectin-2, Sialic acid-containing receptors, $nLc_4Cer$, HSP70/HSP90, GRP78, Laminin receptor and TIM-1.

10. The lipoparticle according to any one of claims 1 to 9, wherein the lipid bilayer further comprises a lipid-bound fluorophore.

11. A method for quantification of Spike mediated binding of the lipoparticle to a cell in a sample comprising the steps:

(a) providing a lipoparticle according to claim 10;
(b) contacting the lipoparticle with a sample, to bind the lipoparticle to the cells contained in the sample;
(c) washing the sample, thereby removing the lipoparticles not bound to the cells:
(d) detecting or determining fluorescence in the washed sample, thereby detecting or determining the number of lipoparticles bound to the cells in the sample.

12. The method according to claim 11, further comprising step (c') before step (c).
(c') detecting or determining fluorescence in the sample, thereby detecting or determining the total number of lipoparticles in the sample.

13. A method for quantification of binding of a lipoparticle to an antibody that specifically binds to the Spike protein in a sample comprising the steps:

(a) providing a lipoparticle according to claim 10;
(b) contacting the lipoparticle with a sample, to bind the synthetic lipoparticle to the antibody contained in the sample;
(c) detecting or determining fluorescence in the sample, thereby detecting or determining an amount of antibody that binds to the lipoparticle in the sample.

14. A method for screening for and/or identifying an agent or molecule that can block or inhibit Spike mediated binding of a synthetic lipoparticle to a cell in a sample comprising the steps:

(a) providing a lipoparticle according to claim 10 having a lipid-bound Spike protein ectodomain, wherein the Spike protein ectodomain binds to a cell surface receptor; and providing a test agent or a test molecule;
(b) mixing or combining the test agent or molecule with a cells containing sample
(c) contacting the lipoparticle with the sample comprising the test agent or molecule, to bind the lipoparticle to the cells contained in the sample;
(d) washing the sample, thereby removing the lipoparticles not bound to the cells;
(e) detecting or determining if the lipoparticles are bound to the cells in the sample by detecting or determining fluorescence in the washed sample.

15. The lipoparticle according to any one of claims 1 to 10, wherein the lipoparticle has a diameter between 80 nm and 140 nm as measured by dynamic light scattering.

# Figure 1

# Figure 2

# Figure 3

# Figure 4

| | | |
|---|---|---|
| **A** | plain SARS-CoV2 lipoparticle | **without** Spike protein |
| **B** | plain Hepatitis B lipoparticle | **without** Spike protein |
| **C** | plain Dengue virus lipoparticle | **without** Spike protein |
| **D** | SARS-CoV2 lipoparticle | **with** SARS-CoV2 (WT) Spike protein |
| **E** | Hepatitis B lipoparticle | **with** HBVAg adw2 protein |
| **F** | Dengue virus lipoparticle | **with** Dengue virus E protein |

# Figure 5

# Figure 5 continued

# Figure 6

Europäisches
Patentamt

European
Patent Office

Office européen
des brevets

# EUROPEAN SEARCH REPORT

Application Number

EP 21 19 3839

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2019/032463 A1 (ICAHN SCHOOL MED MOUNT SINAI [US]; GLAXOSMITHLINE BIOLOGICALS S A [BE]) 14 February 2019 (2019-02-14) * paragraph [0231] – paragraph [0390] * ----- | 1-15 | INV. A61K9/00 A61K9/08 A61K9/127 A61K9/51 |
| Y | BRUNO SAINZ ET AL: "The Aromatic Domain of the Coronavirus Class I Viral Fusion Protein Induces Membrane Permeabilization: Putative Role during Viral Entry &dagger;", BIOCHEMISTRY, vol. 44, no. 3, 1 January 2005 (2005-01-01), pages 947-958, XP055388821, ISSN: 0006-2960, DOI: 10.1021/bi048515g * page 948, right-hand column * ----- | 1-15 | A61K39/12 A61P31/14 A61P31/16 |
| Y | WO 2012/128628 A1 (MUCOSIS BV [NL]; LEENHOUTS CORNELIS JOHANNES [NL] ET AL.) 27 September 2012 (2012-09-27) * claims 1-16 * * page 7, line 16 – page 9, line 16 * ----- | 1-15 | |
| Y | WO 2019/143969 A1 (MALDONADO CLAUDIO [US]; BAUER PHILLIP [US]) 25 July 2019 (2019-07-25) * claims 1-24 * ----- | 1-15 | **TECHNICAL FIELDS SEARCHED (IPC)** A61K A61P |

The present search report has been drawn up for all claims

| Place of search | Date of completion of the search | Examiner |
|---|---|---|
| Munich | 10 March 2022 | Schifferer, Hermann |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 19 3839

This annex lists the patent family members relating to the patent documents cited in the above-mentioned European search report.
The members are as contained in the European Patent Office EDP file on
The European Patent Office is in no way liable for these particulars which are merely given for the purpose of information.

10-03-2022

| Patent document cited in search report | | Publication date | Patent family member(s) | | Publication date |
|---|---|---|---|---|---|
| WO 2019032463 | A1 | 14-02-2019 | NONE | | |
| WO 2012128628 | A1 | 27-09-2012 | AU | 2012231896 A1 | 17-10-2013 |
| | | | BR | 112013024157 A2 | 06-12-2016 |
| | | | CA | 2830901 A1 | 27-09-2012 |
| | | | CN | 103547676 A | 29-01-2014 |
| | | | DK | 2689016 T3 | 27-04-2015 |
| | | | EP | 2689016 A1 | 29-01-2014 |
| | | | ES | 2535421 T3 | 11-05-2015 |
| | | | JP | 6069295 B2 | 01-02-2017 |
| | | | JP | 2014513934 A | 19-06-2014 |
| | | | NZ | 615721 A | 31-07-2015 |
| | | | US | 2014093532 A1 | 03-04-2014 |
| | | | WO | 2012128628 A1 | 27-09-2012 |
| | | | ZA | 201307124 B | 28-05-2014 |
| WO 2019143969 | A1 | 25-07-2019 | AU | 2019210204 A1 | 13-08-2020 |
| | | | EP | 3740211 A1 | 25-11-2020 |
| | | | US | 2020338003 A1 | 29-10-2020 |
| | | | WO | 2019143969 A1 | 25-07-2019 |

EPO FORM P0459

For more details about this annex : see Official Journal of the European Patent Office, No. 12/82

**EP 4 140 475 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- *CHEMICAL ABSTRACTS,* 923585-42-6
- *Nature,* 2020, vol. 581, 221-3, 224